# EUROPEAN PATENT SPECIFICATION

(11) **EP 3 586 834 B1**
(45) Date of publication and mention of the grant of the patent: **02.03.2022**
(21) Application number: 18179396.9
(22) Date of filing: 22.06.2018
(51) Int. Cl.: A61K 31/00, A61K 31/7032, A61P 31/04

(54) **COMPOUND FOR USE IN THE TREATMENT AND/OR PREVENTION OF EPIZOOTIC RABBIT ENTEROPATHY**
VERBINDUNG ZUR BEHANDLUNG UND/ODER VORBEUGUNG VON EPIZOOTISCHER KANINCHENENTEROPATHIE
COMPOSÉ DESTINÉ À ÊTRE UTILISÉ DANS LE TRAITEMENT ET/OU LA PRÉVENTION DE L'ENTÉROPATHIE ÉPIZOOTIQUE DU LAPIN

(43) Date of publication of application: 01.01.2020
(73) Proprietor: HUVEPHARMA, 2600 Antwerpen (BE)
(72) Inventor: PETKOV, Spas, 4550 Peshtera (BG); HAUTEKIET, Veerle, 2600 Antwerpen (BE); VAN GAVER, Davy Philip, 9630 Munkzwalm (BE)
(74) Representative: Gevers Patents

(56) References cited:
- A. MARTEL ET AL: "Susceptibility of Clostridium perfringens strains from broiler chickens to antibiotics and anticoccidials", AVIAN PATHOLOGY, vol. 33, no. 1, 1 February 2004 (2004-02-01), pages 3-7, XP055535487, GB ISSN: 0307-9457, DOI: 10.1080/0307945031000163291
- CHRISTINE BÄUERL ET AL: "Changes in Cecal Microbiota and Mucosal Gene Expression Revealed New Aspects of Epizootic Rabbit Enteropathy", PLOS ONE, vol. 9, no. 8, 22 August 2014 (2014-08-22) , page e105707, XP055535493, DOI: 10.1371/journal.pone.0105707

## Description

### FIELD OF INVENTION

This present invention relates to compounds for use in the treatment and/or prevention of Epizootic Rabbit Enteropathy.

### BACKGROUND OF THE INVENTION

Epizootic Rabbit Enteropathy (ERE) is a highly contagious gastrointestinal syndrome that affects rabbits that are bred in captivity. ERE is a disease that affects mainly young rabbits between six and eight weeks old and also occasionally occurs in suckling or adult rabbits. The term epizootic refers to the spread of a disease within a non-human animal population. In this specific case, high population densities of rabbits being bred contribute to the rapid spread of the disease.

ERE is characterised by an increased morbidity of the affected rabbits. Symptoms include a distended, bloated abdomen (ruminal tympany), caecal impaction, presence of mucus in the colon and excretion thereof, emission of watery droppings or diarrhoea and borborygmus. Accompanying this increased morbid state of the rabbits, a sharp decrease of feed intake and feed conversion is noticed, resulting in a decreased bodyweight gain. Given the highly contagious character of ERE, in a few days all rabbits housed together show symptoms of morbidity. Additionally, a mortality of 30 to 40% is observed, resulting in major economic losses.

ERE is distinct from enterocolitis in other animal species. It is considered an infectious, possibly bacterial, disease although the etiological agent causing ERE has not yet been identified to date (Huybens et al. (2008). Proceedings of the 9th World Rabbit Congress: 971-976). Huybens *et al.* showed that no virus can be considered as the etiological agent of ERE, whereas there are strong indications supporting the hypothesis that ERE has a bacterial etiology. However, the bacteria responsible for causing ERE have not been identified, making it more difficult to find a suited therapy for the syndrome. There are some suggestions that the bacterium might be related to *Clostridum,* but is probably not cultivable. The bacterium causing ERE is aero-tolerant and resistant up to 1 year on a dry medium at room temperature. It produces a toxin that can be neutralized by heat. It was shown that in the rabbit *C. perfringens* type A can be considered non-pathogenic. Moreover, it is commonly accepted that only *C. perfringens* type E and its iota toxin are implicated in the intestinal pathology of rabbits. This toxinotype is however only very rarely isolated in ERE. *C. perfringens* type A is predominantly found in the rabbit and can be considered a microorganism which can colonize the digestive tract of the rabbit and in particular, through influence of certain factors, in rabbits on a protein-rich diet.

In view of the above there is a need for a for a compound for use in the treatment and/or prevention of ERE that may overcome the current problem of increased morbidity and/or mortality in rabbits.

### SUMMARY OF THE INVENTION

The inventors have now surprisingly found that it is possible to fulfill the above mentioned needs by the provision of the compounds of the invention, in particular bambermycin and analogous compounds. While bambermycin and analogous compounds are currently used in poultry, swine and cattle as feed additives and growth promoters, the inventors have surprisingly found that these compounds are effective in preventing and treating ERE.

It is an objective of the present invention to provide compounds to effectively treat and/or prevent ERE. The compounds and methods of the invention allow to reduce the morbidity and/or mortality caused by this syndrome.

The present invention relates to a compound for use in the treatment and/or prevention of Epizootic Rabbit Enteropathy (ERE) wherein said compound is at least one of the compounds according to general formulae (I), (II), (III) or (IV), or the pharmaceutically acceptable salt, pharmaceutically acceptable solvate, or mixture thereof; 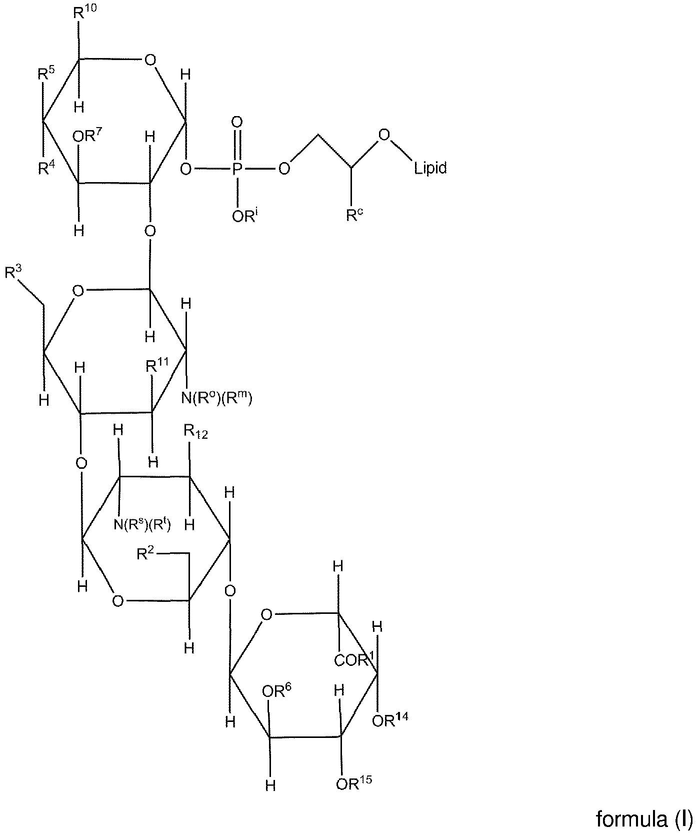 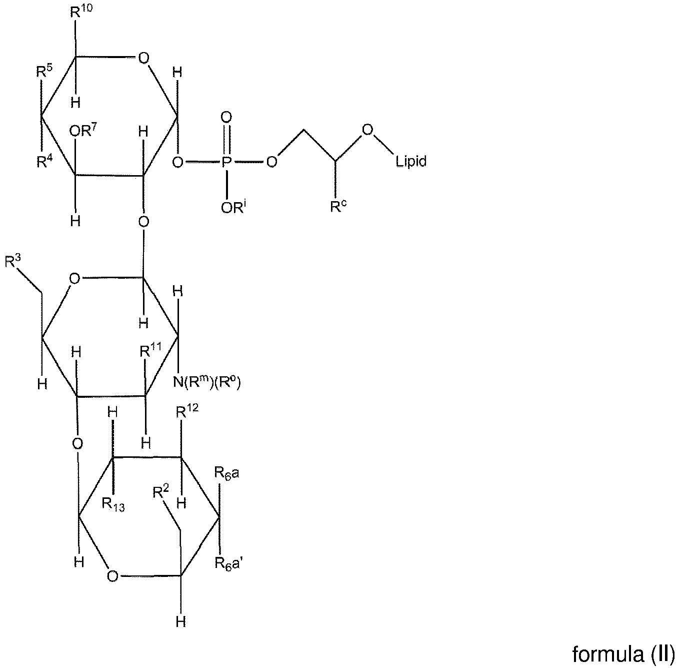 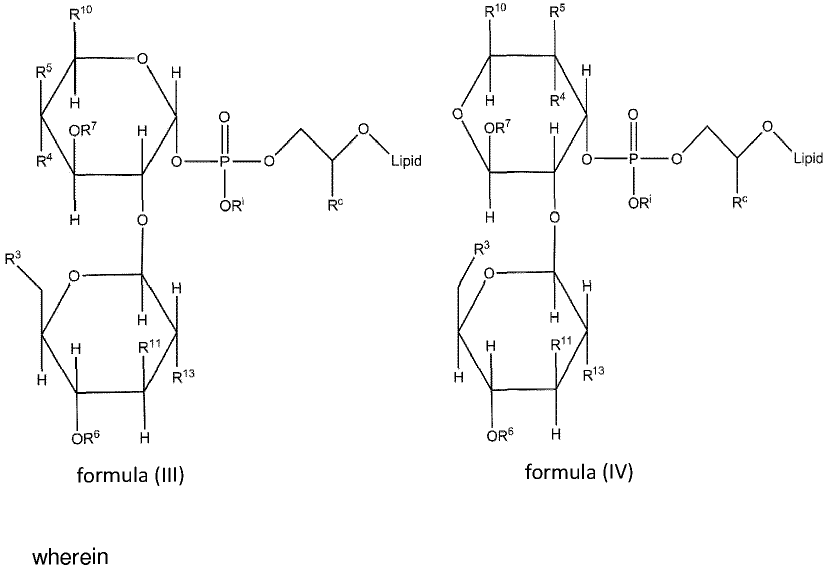
- each of R¹ is independently selected from -OH or -N(R^{1a})(R^{1b}), wherein each of R^{1a} and R^{1b}, independently from each other and at each occurrence, are selected from the group consisting of H, an amino protecting group, aryl, heteroaryl, aliphatic and a heteroaliphatic group,
- each of R², R⁴, R⁵, R¹¹ and R¹², independently from each other and at each occurrence, are selected from the group consisting of H, -OR^{z}, -N(R^{z})₂, aryl, heteroaryl, aliphatic, and a heteroaliphatic group, wherein R^{z} is independently selected from the group consisting of H, an hydroxyl protecting group, an amino protecting group, aryl, heteroaryl, aliphatic, heteroaliphatic and a carbohydrate moiety,
- each of R³ is independently selected from the group consisting of H, -OH, -NH₂, -SH, OR^{w},-NH(R^{w}), -N(R^{w})₂, -SR^{w}, -O(C=O)R^{w}, -NH(C=O)R^{w}, -O(C=NH)R^{w}, -NH(C=NH)R^{w}, -S(C=NH))R^{w},
- NH(C=S)R^{w}, -S(C=O)R^{w}, -O(C=S)R^{w}, -S(=S)R^{w}, aryl, heteroaryl, aliphatic and a heteroaliphatic group, wherein R^{w} is selected from the group consisting of a carbohydrate moiety, aryl, heteroaryl, aliphatic, and heteroaliphatic group,
- each of R⁶, R¹⁴ and R¹⁵, independently from each other and at each occurrence are selected from the group consisting of H, hydroxyl protecting group, aryl, heteroaryl, aliphatic, heteroaliphatic group and carbohydrate moiety,
- each of R⁷ is independently selected from the group consisting of H, C(=O)N(R^{z'})₂, -C(=O)OR^{z'} , an hydroxyl protecting group, aryl, heteroaryl, aliphatic, heteroaliphatic group and carbohydrate moiety, wherein R^{z'} is independently selected from the group consisting of H, an hydroxyl protecting group, an amino protecting group, aryl, heteroaryl, aliphatic, and a heteroaliphatic group,
- each of R¹⁰ is independently selected from the group consisting of -C(=O)N(R^{l})(R^{p}), -C(=O)OR^{k}, and -CH₂OR^{k}, wherein R^{l} and R^{p} are independently from each other and at each occurrence selected from the group consisting of H, an amino protecting group, aryl, heteroaryl, aliphatic, heteroaliphatic group and a carbohydrate moiety, wherein R^{k} is independently selected from the group consisting of H, an hydroxyl protecting group, aryl, heteroaryl, aliphatic, heteroaliphatic group and a carbohydrate moiety,
- each of R¹³ is independently selected from the group consisting of -OH and -N(R^{o"})(R^{m"}) , wherein R^{o"} and R^{m"} independently from each other and at each occurrence, are selected from the group consisting of H, -C(=O)R^{h}, an amino protecting group, aryl, heteroaryl, aliphatic and heteroaliphatic group, wherein R^{h} is selected from the group consisting of a carbohydrate moiety, aryl, heteroaryl, aliphatic and heteroaliphatic group,
- each of (R°) and (R^{m}), independently from each other and at each occurrence, are selected from the group consisting of H, -C(=O)R^{w'}, an amino protecting group, aryl, heteroaryl, aliphatic and heteroaliphatic group, wherein R^{w'} is selected from the group consisting of a carbohydrate moiety, aryl, heteroaryl, aliphatic and heteroaliphatic group;
- each of (R^{s}) and (R^{t}), independently from each other and at each occurrence are selected from the group consisting of H, -C(=O)R^{w"}, an amino protecting group, aryl, heteroaryl, aliphatic and heteroaliphatic group, wherein R^{w"} is selected from the group consisting of a carbohydrate moiety, aryl, heteroaryl, aliphatic and heteroaliphatic group;
- each of R^{6a} and R^{6a'}, independently from each other and at each occurrence are selected from H or -OH, preferably R^{6a} is H and R^{6a'} is -OH;
- each of R^{c} is independently selected from the group consisting of H, halogen, heteroaryl, -OR^{q},
- N(R^{q})₂, -SR^{q}, NO₂, -NC, -CN, -N₃, -N(R^{q})=NR^{q}, -CHO, -C(=O)R^{q}, -C(=S)R^{q}, C(=NR^{q})R^{q},-C(=O)OR^{q}, -C(=NR^{q})OR^{q}, -C(=NR^{q})N(R^{q})₂, -C(=O)N(R^{q})₂, -C(=S)OR^{q}, -C(=O)SR^{q}, -C(=S)SR^{q},-P(=O)(OR^{q})₂, -S(=O)(OR^{q}), -S(=0)₂(OR^{q}), -P(=O)N(R^{q})₂, -P(=O)₂N(R^{q})₂, -C(=0)NR'S(=O)₂R^{q},-S(=O)N(R^{q})₂ and -S(=O)₂N(R^{q})₂, wherein each of R^{q} is independently selected from the group consisting of H, aliphatic, heteroaliphatic, aryl, heteroaryl, and an hydroxyl protecting group,
- each of Rⁱ is independently selected from the group consisting of H, an hydroxyl protecting group, aliphatic, heteroaliphatic, aryl, and heteroaryl,
- each Lipid is independently selected from H or a C₁₋₃₀ aliphatic moiety, wherein 0 to 10 methylene units are optionally replaced with -O-, -NR^{x}-, -S-, -C(=O)-, -C(=NR^{x})-, -S(=O)-,-S(=O)₂-, -N=N-, -C=N-, -C(R^{y})=C(R^{y'})-, -N-O-, an arylene, or an heteroarylene moiety, wherein each of R^{x} is independently selected from the group consisting of H, aliphatic, heteroaliphatic, aryl, heteroaryl, or an amino protecting group, and wherein each of R^{y} and R^{y'}, independently from each other and at each occurrence are selected from the group consisting of H, aliphatic, heteroaliphatic, aryl and heteroaryl group.

The present invention further relates to a compound for use in the treatment and/or prevention of ERE comprising administering 0.5 to 100 mg of the compound as defined in any one of the embodiments herein.

The present invention further relates to a compound, as defined in any one of the embodiments presented herein, for use in the treatment and/or prevention of ERE by oral administration of the compound.

The present invention further relates to a composition for use in the treatment and/or prevention of ERE, the composition comprising the compound as defined in any one of the embodiments presented herein.

The present invention further relates to a composition for use in the treatment and/or prevention of ERE, the composition comprising bambermycin.

The present invention further relates to a composition for use in the treatment and/or prevention of ERE, the composition comprising 1 to 100 ppm of the compound.

### BRIEF DESCRIPTION OF THE DRAWINGS

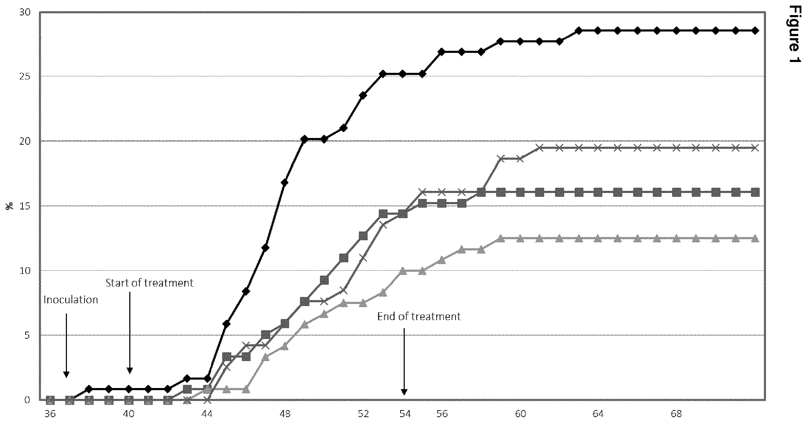

Figure 1 shows the ERE-related cumulative mortality rate combining all rabbits, both inoculated and test rabbits. The control group (rotated squares) consistently showed the highest mortality rates. The group of rabbits treated with 16ppm bambermycin (squares), 24ppm bambermycin (triangles) or 32ppm bambermycin (crosses) show a clearly reduced mortality rate evolution.

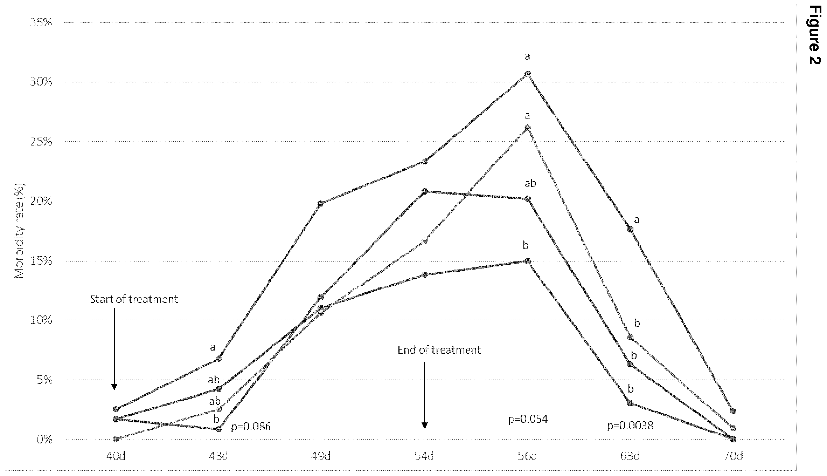

Figure 2 shows the evolution of the morbidity rate for the control group and three treatment groups. Based on the individual clinical observations, the highest incidence of animals demonstrating clinical signs of ERE was observed at 56 days of age in the control group. Overall, untreated control animals showed the highest morbidity during and after the treatment period, although not always significant.

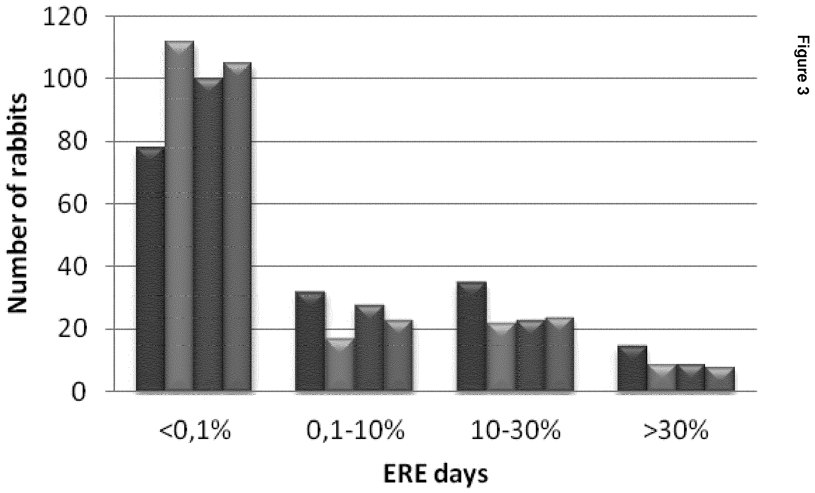

Figure 3 shows for each group (either treatment or control) the number of rabbits for which a certain amount of days with ERE symptoms were registered. For each of the four time intervals, each first vertical bar represents the amount of animals of the control group, whereas the following three bars represent the amount of animals of the groups receiving feed supplemented with, respectively, 16ppm bambermycin, 24ppm bambermycin and 32ppm bambermycin. The animals receiving feed supplemented with bambermycin had significantly (P <0.01) less days with ERE (Least Square Mean (LSM) of 5.6% days) compared to the control group (LSM of 9.1%).

### DETAILED DESCRIPTION OF THE INVENTION

It is further understood that all definitions and preferences as described for the compounds of the invention above equally apply for this embodiment and all further embodiments, as described below.

As used in the foregoing and hereinafter, the following definitions apply unless otherwise noted.

A "moenomycin" as used herein refers to a member of the moenomycin family of antibiotics. Moenomycins are phosphoglycolipid antibiotics, originally obtained from *Streptomyces.* The most notable moenomycins, and those preferred in the present invention, include moenomycin A, moenomycin A₁₂, moenomycin C₁, moenomycin C₃, moenomycin C₄, pholipomycin, AC326-alpha, nosokomycin A, nosokomycin B, nosokomycin C, and nosokomycin D.

"Bambermycin" is a known complex of compounds, primarily composed of moenomycins A and C. It can be obtained from *Streptomyces bambergiensis* and *Streptomyces ghanaensis Streptomyces ederensis, Streptomyces geysiriensis* and related strains or biosynthesized. Bambermycin is also known as flavophospholipol and is available under its commercial name Flavomycin or Gainpro. Bambermycin, flavophospholipol and Flavomycin are used interchangeably herein.

"Ppm" and percentages as used herein refer to mass fractions, unless its context clearly dictates otherwise.

The term "aliphatic" includes both saturated and unsaturated, nonaromatic, straight chain (i.e., unbranched), branched, acyclic, and cyclic hydrocarbon chain having 1 to 30 carbon atoms, which are optionally substituted with one or more groups including but not limited to alkyl, alkynyl, alkenyl, aryl, halide, nitro, amino, ester, ketone, aldehyde, hydroxy, carboxylic acid, or alkoxy. According to certain embodiments, as used herein, C_{F-G} aliphatic defines a C_{F-G} aliphatic group having F to G carbon atoms, e.g. C₁₋₁₂ aliphatic defines an aliphatic group containing 12 carbon atoms, C₁₋₁₀ aliphatic defines an aliphatic group containing 1 to 10 carbon atoms.

As will be appreciated by one of ordinary skilled in the art, "aliphatic" is intended herein to include, but is not limited to alkyl, alkenyl, and alkynyl moieties. As used herein the term "alkyl" "alkenyl", and "alkynyl" have the broadest meaning generally understood in the art, and may include a moiety which is linear, branched, cyclic (cycloalkyl, cycloalkenyl, cycloalkynyl) or a combination thereof.

The term "alkyl", alone or in combination means an alkane-derived radical, which may be a straight chain alkyl, branched alkyl or cyclic alkyl, containing from 1 to 30 carbon atoms, unless otherwise specified. The straight chain or branched alkyl group is attached at any available point to produce a stable compound. Alkyl also includes a straight chain or branched alkyl group that contains or is interrupted by a cycloalkyl portion. According to certain embodiments C_{A-B} alkyl defines a straight or branched alkyl radical having from A to B carbon atoms, e.g. C₁₋₁₅ alkyl defines a straight or branched alkyl radical having from 1 to 15 carbon atoms, C₁₋₁₂ alkyl defines a straight or branched alkyl radical having from 1 to 12 carbon atoms, C₁₋₆ alkyl defines a straight or branched alkyl radical having from 1 to 6 carbon atoms such as for example methyl, ethyl, 1-propyl, 2-propyl, I-butyl, 2-butyl, 2-methyl-1-propyl. According to certain embodiments a cyclic C_{C-D}alkyl defines a cyclic alkyl radical having from C to D carbon atoms, e.g. C₃₋₆ cyclic alkyl.

The term "alkenyl", alone or in combination, means a straight or branched hydrocarbon containing 1-30 carbon atoms, unless otherwise specified and at least one carbon to carbon double bond. Examples of alkenyl groups include ethenyl, propenyl, isopropenyl, butenyl, cyclohexenyl, cyclohexenylalkyl and the like. Alkenyl also includes a straight chain or branched alkenyl group that contains or is interrupted by a cycloalkyl portion. Carbon to carbon double bonds may be either contained within a cycloalkyl portion or within a straight chain or branched portion. According to certain embodiments C_{H-I} alkenyl defines a straight or branched alkenyl radical having from H to I carbon atoms, e.g. C₁₋₆ alkenyl defines a straight or branched alkenyl radical having from 1 to 6 carbon atoms.

The term "alkynyl" alone or in combination means a straight, branched or cyclic hydrocarbon containing 1 to 30 carbon atoms containing at least one carbon to carbon triple bond. Examples of alkynyl groups include ethynyl, propynyl, butynyl and the like.

As used herein, the term "heteroaliphatic," refers to an aliphatic moiety, as defined herein, which includes both saturated and unsaturated, nonaromatic, straight chain (i.e.,unbranched), branched, acyclic, cyclic (i.e., heterocyclic), or polycyclic hydrocarbons having 1 to 30 carbon atoms, unless specified otherwise, which are optionally substituted with one or more groups, including but not limited to alkyl, alkynyl, alkenyl, aryl, halide, nitro, amino, ester, ketone, aldehyde, hydroxy, carboxylic acid, or alkoxy and that contain one or more hetero atoms such as oxygen, sulfur, nitrogen, phosphorus, or silicon atoms, e.g., in place of carbon atoms. In certain embodiments, heteroaliphatic moieties are substituted by independent replacement of one or more of the hydrogen atoms thereon with one or more substituents. As will be appreciated by one of ordinary skilled in the art, "heteroaliphatic" is intended herein to include heteroalkyl, heterocycloalkyl, heterocycloalkenyl, and heterocycloalkynyl moieties. Thus, as used herein, the term "heteroalkyl" includes straight, branched and cyclic alkyl groups that contain one or more one or more hetero atoms such as oxygen, sulfur, nitrogen, phosphorus, or silicon atoms, e.g., in place of carbon atoms. An analogous convention applies to other generic terms such as "heteroalkenyl", "heteroalkynyl", and the like. Furthermore, as used herein, the terms "heteroalkyl", "heteroalkenyl", "heteroalkynyl", and the like encompass both substituted and unsubstituted groups.

As used herein, the term "aryl", alone or in combination means any carbon-based aromatic group including, but not limited to, phenyl, tolyl, xylyl, cumenyl, naphthyl, anthracenyl etc., optionally carbocyclic fused with a cycloalkyl or heterocyclyl of preferably 5-7, more preferably 5-6, ring members and/or optionally substituted with 1 to 5 groups or substituent. An aryl may be optionally substituted with one or more groups including but not limited to alkyl, alkynyl, alkenyl, aryl, halide, nitro, amino, ester, ketone, aldehyde, hydroxy, carboxylic acid, or alkoxy whereby the substituent is attached at one point to the aryl or whereby the the substituent is attached at two points to the aryl to form a bicyclic system e.g. benzodioxole, benzodioxan, benzimidazole.

The term "heteroaryl", alone or in combination means a monocyclic aromatic ring structure containing 5 or 6 ring atoms, or a bicyclic aromatic group having 8 to 10 atoms, containing one or more, preferably 1-4, more preferably 1-3, heteroatoms independently selected from the group O, S, and N, and optionally substituted with 1 to 5 groups or substituents including but not limited alkyl, alkynyl, alkenyl, aryl, halide, nitro, amino, ester, ketone, aldehyde, hydroxy, carboxylic acid, or alkoxy Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. More specifically the term heteroaryl includes, but is not limited to, pyridyl, furanyl, thiophenyl, thiazolyl, isothiazolyl, triazolyl, imidazolyl, isoxazolyl, pyrrolyl, pyrazolyl, pyrimidinyl, benzofuranyl, isobenzofuranyl, benzothiazolyl, benzoisothiazolyl, benzotriazolyl, indolyl, isoindolyl, benzoxazolyl, quinolyl, isoquinolyl, benzimidazolyl, benzisoxazolyl, benzothiophenyl, dibenzofuran, and benzodiazepin-2-one-5-yl, and the like.

According to a certain embodiment of the present invention, the Lipid is an unsaturated C₄₋₂₀ hydrocarbon chain, wherein 1 to 10 methylene units are replaced by-C(R^{y})=C(R^{y'})- wherein each of R^{y} and R^{y'}, independently from each other and at each occurrence, are selected from the group consisting of H, aliphatic, heteroaliphatic, aryl, or heteroaryl; and wherein said hydrocarbon chain is optionally substituted with a C₁₋₆ alkyl, a C₃₋₆ cyclic alkyl, C₁₋₆ alkenyl or hydroxyl group, preferably the Lipid is a C₄₋₂₀ hydrocarbon chain, wherein 1 to 5 methylene units are replaced by -C(R^{y})=C(R^{y'})-wherein each of R^{y} and R^{y'}, independently from each other and at each occurrence, are selected from the group consisting of H, C₁₋₆ alkyl and C₁₋₆ alkenyl and wherein said hydrocarbon chain is optionally substituted with a C₁₋₆ alkyl, a C₃₋₆ cyclic alkyl, C₁₋₆ alkenyl or hydroxyl group, more preferably the Lipid is chosen among following formulae: 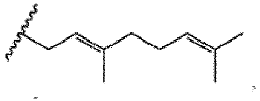 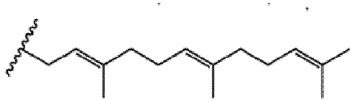 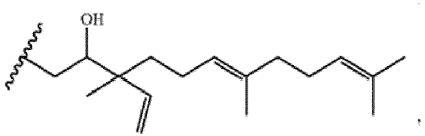 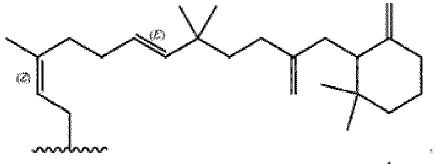 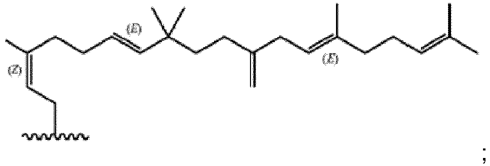 and

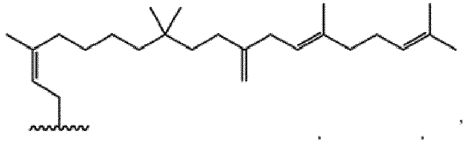

Even more preferred, the Lipid is chosen among following formulae:

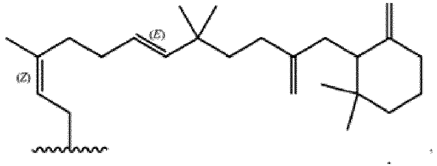

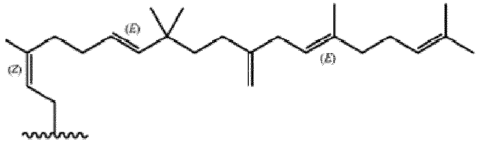

According to a certain embodiment of the present invention, the Lipid is a saturated hydrocarbon chain according to general formula Z: 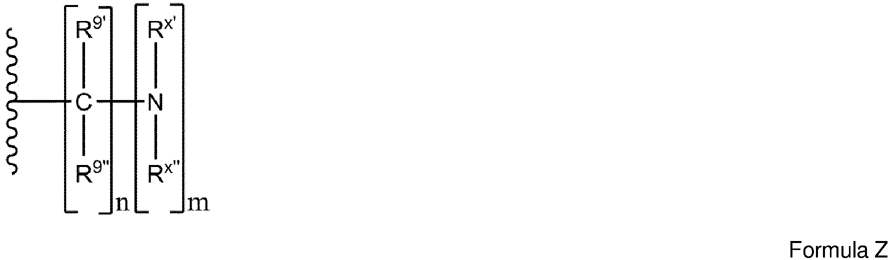 wherein
- each of R^{9'} and R^{9"} are independently from each other and at each occurrence selected from the group consisting of H, -OH and C₁₋₆ alkyl,
- each of R^{x'} and R^{x"} , independently from each other and at each occurrence, are selected from H, a C₁₋₁₅ alkyl and an aryl wherein said aryl is optionally further substituted by a C₁₋₁₅ alkyl, and wherein n = 4 to 30, and m = 0 or 1

Preferably, the saturated hydrocarbon chain is chosen among following formulae: 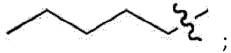 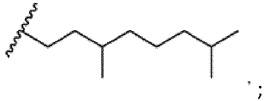 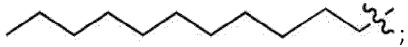 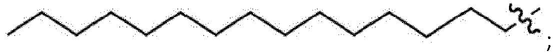 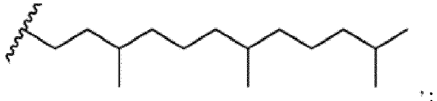 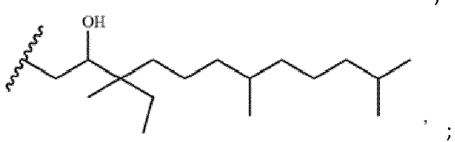 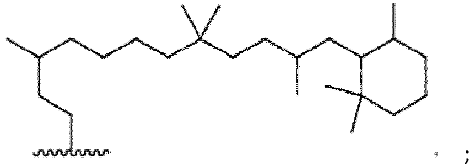 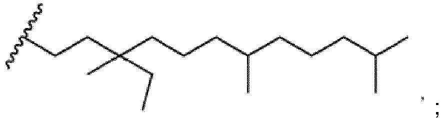 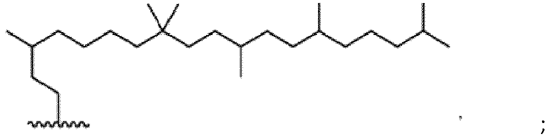 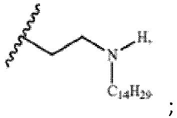 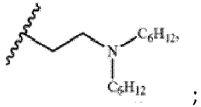 and

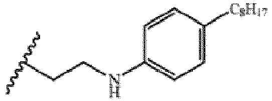

According to a certain embodiment of the present invention, R¹ is independently selected from -OH or -N(R^{1a})(R^{1b}), wherein each of R^{1a} and R^{1b}, independently from each other and at each occurrence, are selected from the group consisting of H, C₁₋₂₀ aliphatic and C₁₋₂₀ heteroaliphatic group, preferably R¹ is OH, NH₂, or NH(R^{1b}), wherein R1b is a C₁₋₁₀ cycloalkyl group or C₁₋₁₀ cycloalkenyl which is optionally substituted with an halide, hydroxyl or alkoxy, more preferably R¹ is -NH(R^{1b}), wherein R^{1b} is of following formula:

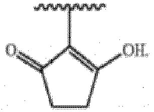

According to a certain embodiment of the present invention, R³ is independently selected from the group consisting of H, -OH and -OR^{w}, wherein R^{w} is selected from the group consisting of a carbohydrate moiety, wherein said carbohydrate moiety is independently selected from the group consisting of D- and L- monosaccharides, such as notably D-erythrose, L-erythrose, D-threose, L-threose, L- erythrulose, D- erythrulose, D-arabinose, L-arabinose, D- deoxyribose, L- deoxyribose, D-lyxose, L-lyxose, D-ribose, L-ribose, D-ribulose, L-ribulose, D-xylose, L-xylose, D-xylulose, L-xylulose, D-allose, L- allose, D-altrose, L-altrose, D-galactose, L-galactose, D-glucose, L-glucose, D-gulose, L-gulose, D-idose, L-idose, D-mannose, L-mannose, D-talose, L-talose, D-fructose, L-fructose, D-psicose, L-psicose, D-sorbose, L-sorbose, D-tagatose, L-tagatose, D-fucose, L-fucose, D-rhamnose and L-rhamnose, disaccharides, such as notably sucrose, lactose, trehalose, and maltose, trisaccharides such as notably acarbose, raffinose, and melezitose, more preferably, R³ is -OR^{w}, wherein R^{w} is a D-monosaccharide such as notably D-erythrose, D-threose, D- erythrulose, D-arabinose, D-deoxyribose, D-lyxose, D-ribose, D-ribulose, D-xylose, D-xylulose, D-allose, D-altrose, D-galactose, D-glucose, D-gulose, D-idose, D-mannose, D-talose, D-fructose, D-psicose, D-sorbose, D-tagatose, D-fucose, and D-rhamnose, even more preferably, R³ is -OR^{w}, wherein R^{w} is independently selected from the group consisting of D-glucose, D-galactose, D-allose, D-altrose, D-mannose, D-iodose, D-galose or D-talose, most preferably, R³ is -OR^{w}, wherein R^{w} is D-glucose.

According to a certain embodiment of the present invention, each of R², R⁴, R⁵, R¹¹ and R¹², independently from each other and at each occurrence, are selected from the group consisting of H, OR^{z}, a C₁₋₁₂ aliphatic, C₁₋₁₂ heteroaliphatic and a carbohydrate moiety, wherein R^{z} is selected from the group consisting of H, hydroxyl protecting group, C₁₋₁₂ aliphatic, C₁₋₁₂ heteroaliphatic, aryl and heteroaryl, and wherein said carbohydrate moiety is selected from the group consisting of D-monosaccharides such as notably D-erythrose, D-threose, D- erythrulose, D-arabinose, D-deoxyribose, D-lyxose, D-ribose, D-ribulose, D-xylose, D-xylulose, D-allose, D-altrose, D-galactose, D-glucose, D-gulose, D-idose, D-mannose, D-talose, D-fructose, D-psicose, D-sorbose, D-tagatose, D-fucose, and D-rhamnose, preferably each R², R⁴, R⁵, R¹¹ and R¹², independently from each other and at each occurrence, are selected from the group consisting of H, OR^{z}, a hydroxyl protecting group, C₁₋₆ alkyl and a carbohydrate moiety, wherein R^{z} is selected from the group consisting of H, a hydroxyl protecting group and C₁₋₆ alkyl, and wherein said carbohydrate moiety is selected from the group consisting of D-glucose, D-galactose, D-allose, D-altrose, D-mannose, D-iodose, D-galose and D-talose, more preferably R², R⁴, R¹¹ and R¹², independently from each other and at each occurrence, are selected from H or OR^{z}, wherein R^{z} is selected from H or C₁₋₆ alkyl, more preferably R⁵ is a C₁₋₆ alkyl, even more preferably R² is H, R⁴, R¹¹ and R¹² are -OH and R⁵ is -CH₃;

According to a certain embodiment of the present invention, each of R⁶, R¹⁴ and R¹⁵, independently from each other and at each occurrence, are selected from the group consisting of H, hydroxyl protecting group, C₁₋₁₂ aliphatic and C₁₋₁₂ heteroaliphatic group, preferably R⁶, R¹⁴ and R¹⁵, independently from each other and at each occurrence, are selected from the group consisting of H or an hydroxyl protecting group, more preferably R⁶, R¹⁴ and R¹⁵, independently from each other and at each occurrence, are H.

According to a certain embodiment of the present invention, R⁷ is -C(=O)N(R^{z'})₂ group, wherein each of R^{z'} is independently selected from the group consisting of R^{z'} is selected from the group consisting of H, aryl, heteroaryl and C₁₋₁₂ alkyl, preferably each of R^{z'} is independently selected from H or C₁₋₁₂ alkyl, more preferably each of R^{z'} is independently selected from the group consisting of H, methyl, ethyl and propyl, most preferably each of R^{z'} is independently H.

According to a certain embodiment of the present invention, each of R¹⁰ is independently selected from-C(=O)N(R^{l})(R^{p}) or -C(=O)OR^{k}, wherein R^{l} and R^{p} are independently from each other and at each occurrence, selected from the group consisting of H, an amino protecting group, aryl, heteroaryl, C₁₋₁₂ aliphatic and C₁₋₁₂ heteroaliphatic group , and wherein R^{k} is independently selected from the group consisting of H, an hydroxyl protecting group, aryl, heteroaryl, C₁₋₃₀ aliphatic and C₁₋₃₀ heteroaliphatic group, preferably each of R¹⁰ is independently selected from -C(=O)N(R^{l})(R^{p}) or-C(=O)OR^{k}, wherein R^{l} and R^{p} are independently from each other and at each occurrence, selected from the group consisting of H, an amino protecting group, aryl, heteroaryl and C₁₋₆ alkyl, wherein each of R^{k} is independently selected from the group consisting of H, an hydroxyl protecting group and C₁₋₆ alkyl, more preferably, each of R¹⁰ is independently selected from -C(=O)N(R^{l})(R^{p}) or-C(=O)OR^{k}, wherein R^{l} and R^{p}, independently from each other and at each occurrence, are selected from the group consisting of H, an amino protecting group, methyl, ethyl, and propyl, wherein each of R^{k} is independently selected from the group consisting of H, an hydroxyl protecting group, methyl, ethyl, and propyl, most preferably, R¹⁰ is -C(=O)NH₂ or -C(=O)OH.

According to a certain embodiment of the present invention, each of R¹³ is independently selected from the group consisting of -OH and -N(R^{o"})(R^{m"}), wherein R^{o"} and R^{m"}, independently from each other and at each occurrence, are selected from the group consisting of H, -C(=O)R^{h}, an amino protecting group, C₁₋₁₂ aliphatic and C₁₋₁₂ heteroaliphatic group, wherein R^{h} is independently selected from a C₁₋₁₂ aliphatic or C₁₋₁₂ heteroaliphatic group, preferably, R^{o"} and R^{m'}, independently from each other and at each occurrence, are selected from the group consisting of H, -C(=O)R^{h} and C₁₋₁₂ alkyl, wherein R^{h} is C₁₋₁₂ alkyl, more preferably R^{o"} and R^{m"}, independently from each other and at each occurrence, are selected from the group consisting of H, -C(=O)R^{h} and C₁₋₆ alkyl, wherein R^{h} is C₁₋₆ alkyl, most preferably R^{o"} is H and R^{m'} is -C(=O)CH₃.

According to a certain embodiment of the present invention, each of (R°) and (R^{m}), independently from each other and at each occurrence, are selected from the group consisting of H, -C(=O)R^{w'}, an amino protecting group, C₁₋₁₂ aliphatic and C₁₋₁₂ heteroaliphatic group, wherein R^{w'} is independently selected from the group consisting of a C₁₋₁₂ aliphatic and C₁₋₁₂ heteroaliphatic group, preferably (R°) and (R^{m}), independently from each other and at each occurrence, are selected from the group consisting of H, -C(=O)R^{w'}, an amino protecting group and C₁₋₁₂ alkyl, wherein R^{w'} is a C₁₋₁₂ alkyl, more preferably (R°) and (R^{m}), independently from each other and at each occurrence, are selected from the group consisting of H, -C(=O)R^{w'}, and C₁₋₆ alkyl, wherein R^{w'} is a C₁₋₆ alkyl, most preferably (R°) is H and (R^{m}) is -C(=O)CH₃.

According to a certain embodiment of the present invention, each of (R^{s}) and (R^{t}), independently from each other and at each occurrence are selected from the group consisting of H, -C(=O)R^{w"}, an amino protecting group, C₁₋₁₂ aliphatic and C₁₋₁₂ heteroaliphatic group, wherein R^{w"} is independently selected from a C₁₋₁₂ aliphatic or C₁₋₁₂ heteroaliphatic group, preferably (R^{s}) and (R^{t}), independently from each other and at each occurrence, are selected from the group consisting of H, -C(=O)R^{w"}, an amino protecting group and C₁₋₁₂ alkyl, wherein R^{w"} is C₁₋₁₂ alkyl, more preferably (R^{s}) and (R^{t}), independently from each other and at each occurrence, are selected from the group consisting of H, -C(=O)R^{w'}, and C₁₋₆ alkyl, wherein R^{w"} is C₁₋₆ alkyl, most preferably (R^{s}) is H and (R^{t}) is -C(=O)CH₃.

According to a certain embodiment of the present invention, each of R^{c} is independently selected from the group consisting of H, -C(=O)OR^{q}, -C(=O)SR^{q}, -C(=S)OR^{q}, -C(=O)SR^{q}, -C(=S)SR^{q}, wherein each of R^{q} is independently selected from the group consisting of H, C₁₋₁₂ alkyl, aryl, heteroaryl and an hydroxyl protecting group, preferably each of R^{c} is independently selected from the group consisting of H, -C(=O)OR^{q}, -C(=O)SR^{q}, and -C(=O)SR^{q}, wherein each of R^{q} is independently selected from the group consisting of H, C₁₋₆ alkyl and an hydroxyl protecting group, more preferably each of R^{c} is independently selected from H or -C(=O)OR^{q}, wherein each of R^{q} is independently selected from the group consisting of H, methyl, ethyl and propyl, most preferably R^{c} is -C(=O)OH.

According to a certain embodiment of the present invention, each of Rⁱ is independently selected from the group consisting of H, an hydroxyl protecting group, C₁₋₁₂ aliphatic, C₁₋₁₂ heteroaliphatic, aryl and heteroaryl, preferably Rⁱ is independently selected from the group consisting of H, an hydroxyl protecting group, C₁₋₁₂ alkyl, aryl and heteroaryl, more preferably Rⁱ is independently selected from the group consisting of H, an hydroxyl protecting group and C₁₋₆ alkyl, even more preferably Rⁱ is independently selected from the group consisting of H, an hydroxyl protecting group, methyl, ethyl, and propyl, most preferably Rⁱ is H.

The compounds for use of the present invention, as detailed above, have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the anti-inflammatory compound as specified herein, may possess.

In a preferred embodiment, the compound of the invention is a moenomycin. Therefore, the present invention provides a moenomycin for use in the prevention and/or treatment of ERE. Preferred compounds for use in the invention are compounds according to formula (Ia) [compounds for use of class I, herein after] or the pharmaceutically acceptable salt, pharmaceutically acceptable solvate, or mixture thereof, 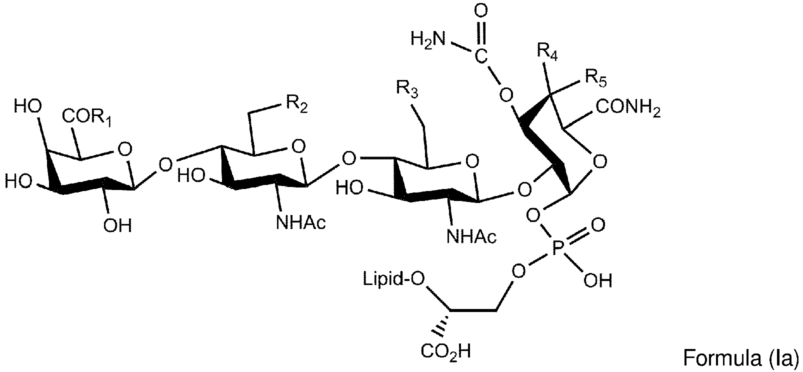 wherein
- each of R¹ is independently selected from OH, NH₂, or NH(R^{1b}), wherein R1b is a C1-10 cycloalkyl group or C1-10 cycloalkenyl which is optionally substituted with an halide, hydroxyl or alkoxy, more preferably R1 is -NH(R^{1b}), wherein R^{1b} is of following formula: 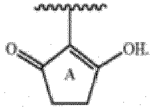
- each of R³ is independently selected from the group consisting of H, -OH and -OR^{w}, wherein R^{w} is D-glucose;
- each of R² and R⁴, independently from each other and at each occurrence are selected from H or -OH;
- each of R⁵ is independently selected from the group consisting of H, -OH and -CH₃;
- the Lipid is chosen among following Formula L₁ or Formula L₂:

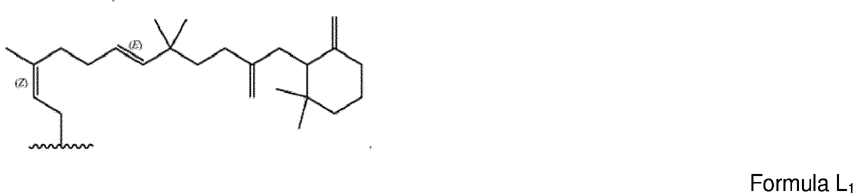

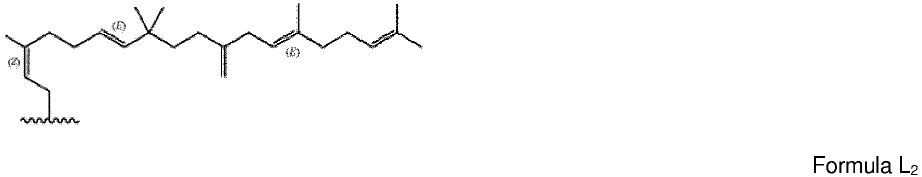

More preferably, the present invention provides a composition for use, wherein the composition comprises a mixture of compounds of class I, as detailed above.

Non limitative examples of compositions for use suitable for the invention include bambermycin, commercially available as Flavomycin^{®}.

A preferred compound for use of class I is moenomycin A according to formula (lb), or the pharmaceutically acceptable salt, pharmaceutically acceptable solvate, or mixture thereof:

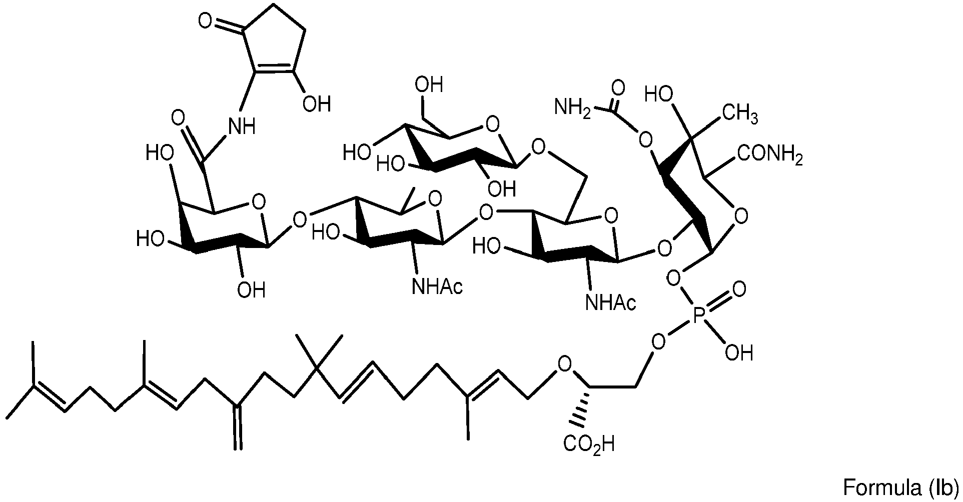

According to an alternative embodiment of the present invention, compounds for use are compounds according to formula (IIa) [compounds for use of class II, herein after] or the pharmaceutically acceptable salt, pharmaceutically acceptable solvate, or mixture thereof: 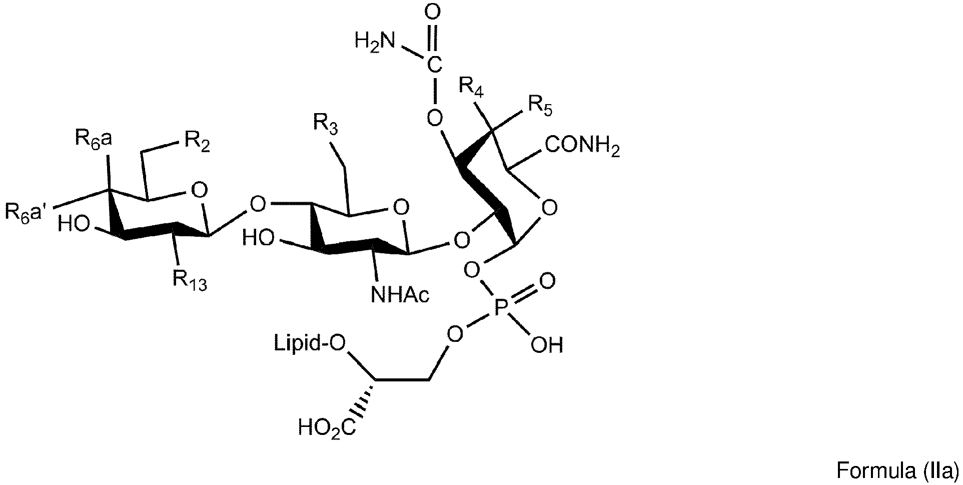 wherein
- R3 is H or -OH;
- each of R² and R⁴, independently from each other and at each occurrence, are selected from H or -OH;
- each of R^{6a} and R^{6a}, independently from each other and at each occurrence, are selected from H or -OH, preferably R^{6a} is H and R^{6a'} is -OH;
- R⁵ is H, -OH or CH₃;
- R¹³ is -OH or -NHC(=O)CH₃
- the Lipid is chosen among following Formula L₃, Formula L₄ or Formula L₅:

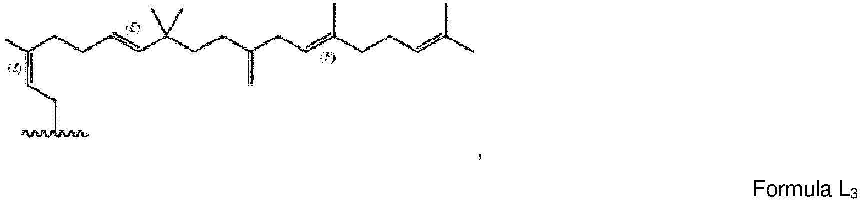

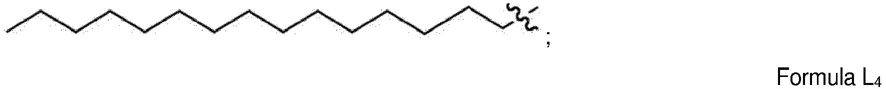

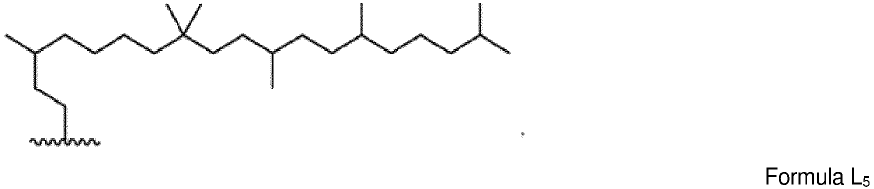

According to an alternative embodiment of the present invention, compounds for use according to the invention are compounds according to formula (IIIa) [compounds for use of class III, herein after] or the pharmaceutically acceptable salt, pharmaceutically acceptable solvate, or mixture thereof: 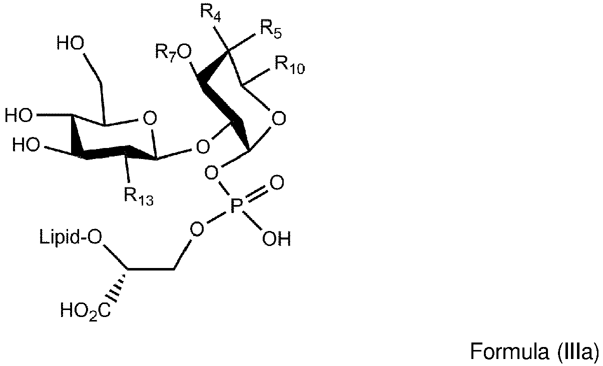 wherein
- each of R⁴ and R⁵, independently from each other and at each occurrence are selected from the group consisting of H, -CH₃ and -OH;
- R₇ is independently selected from H or -C(=O)NH₂, preferably, R₇ is C(=O)NH₂;
- R¹⁰ is independently selected from -C(=O)NH₂ or -C(=O)OH, preferably, R¹⁰ is C(=O)NH₂.;
- R¹³ is independently selected from the group consisting of -OH or -NHC(=O)CH₃, preferably R¹³ is --NHC(=O)CH₃
- the Lipid is chosen among following Formula L₆, Formula L₇ or Formula L₈:

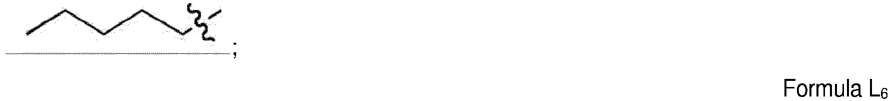

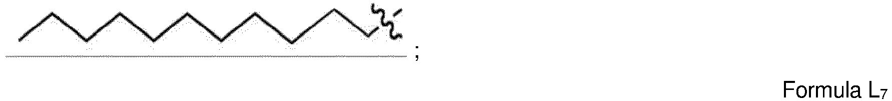

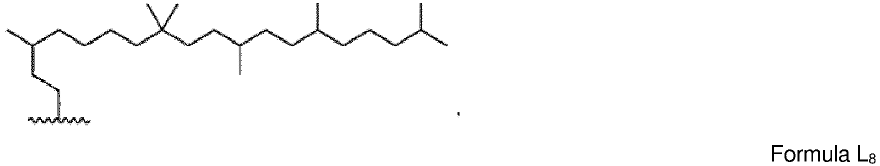

For therapeutic use, salts of the compounds for use of the present invention, as detailed above, are those wherein the counter-ion is pharmaceutically acceptable, which salts can be referred to as pharmaceutically acceptable acid and base addition salts. However, salts of acids and bases that are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable acid and base addition salts as mentioned hereinabove are meant to comprise the therapeutically active non-toxic acid and base addition salt forms that the compounds for use of the present invention, as detailed above, are able to form. The pharmaceutically acceptable acid addition salts can conveniently be obtained by treating the base form with such appropriate acid in an anion form. Appropriate anions comprise, for example, trifluoroacetate, acetate, benzenesulfonate, benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, triethiodide, and the like. The counterion of choice can be introduced using ion exchange resins. Conversely said salt forms can be converted by treatment with an appropriate base into the free base form.

The compounds for use as specified herein, containing an acidic proton may also be converted into their nontoxic metal or amine addition salt forms by treatment with appropriate organic and inorganic bases in a cation form. Appropriate basic salts comprise those formed with organic cations such as benzathine, chloroprocaine, choline, diethanolamine, ethylenediamine, meglumine, procaine, and the like; and those formed with metallic cations such as aluminum, calcium, lithium, magnesium, potassium, sodium, zinc, and the like. Conversely said salt forms can be converted by treatment with an appropriate acid into the free form.

The term addition salt as used hereinabove also comprises the solvates which the compounds for use, as specified herein, as well as the salts thereof, are able to form. Such solvates are for example hydrates, alcoholates and the like.

### COMPOSITIONS

The present invention further relates to a composition for use in the treatment and/or prevention of ERE, the composition comprising a compound as defined above and as defined in any one of the embodiments presented herein, in particular the compound for use of any of the classes (I) to (III), as defined above.

In the rest of the text, the expression "compound " or "compound according to the invention" is understood, for the purposes of the present invention, both in the plural and the singular, that is to say that the inventive composition may comprise one or more than one "compound according to the invention".

In a particular embodiment, the present invention provides moenomycin A, as well as compositions comprising moenomycin A, for use in the prevention and/or treatment of ERE. As a further embodiment of the present invention, the compound according to the invention is the compound with the International Union of Pure and Applied Chemistry (IUPAC) name (2S,3S,4R,5R,6R)-5-[(2S,3R,4R,5S,6R)-3-acetamido-5-[(2S,3R,4R,5S,6R)-3-acetamido-4-hydroxy-6-methyl-5-[(2R,3R,4S,5R,6S)-3,4,5-trihydroxy-6-[(2-hydroxy-5-oxocyclopenten-1-yl)carbamoyl]oxan-2-yl]oxyoxan-2-yl]oxy-4-hydroxy-6-[[(2R,3R,4S,5S,6R)-3,4,5-trihydroxy-6-(hydroxymethyl)oxan-2-yl]oxymethyl]oxan-2-yl]oxy-4-carbamoyloxy-6-[[(2R)-2-carboxy-2-[(2E,6E,13E)-3,8,8,14,18-pentamethyl-11-methylidenenonadeca-2,6,13,17-tetraenoxy]ethoxy]-hydroxyphosphoryl]oxy-3-hydroxy-3-methyloxane-2-carboxylic acid with CAS number 11015-37-5.The present invention further relates to a composition for use, the composition comprising bambermycin. Bambermycin can be obtained from *Streptomyces bambergiensis Streptomyces ghanaensis Streptomyces ederensis, Streptomyces geysiriensis* and related strains. Bambermycin is also known as flavophospholipol. The extraction of bambermycin is e.g. disclosed in GB1139589 and GB1068639.

In said references, bambermycin is referred to as "the antibiotic Moenomycin". Bambermycin is a complex of compounds, primarily composed of moenomycins A and C. Moenomycins are classified as phosphoglycolipids based on their structural composition. They contain 3-phosphoglyceric acid, a unique structural element among bacterial secondary metabolites.

As an embodiment of the present invention, the composition comprising the compound of the present invention as used herein is an antibiotic moenomycin-containing complex obtained from cultures of *Streptomyces bambergiensis, Streptomyces ghanaensis, Streptomyces ederensis, Streptomyces geysiriensis* and related strains. In a particular embodiment, the composition of the invention comprises at least 5 active components, namely moenomycins A, A₁₂, C₁, C₃ and C₄; preferably moenomycin A being the major component. In another embodiment, the composition of the invention comprises at least 800 mg/g moenomycins. In a further embodiment, said composition comprises:
- at least 50% moenomycin A, preferably at least 65%;
- less than 15% moenomycin A₁₂, preferably less than 10%;
- less than 15% moenomycin C₁, preferably less than 10%;
- less than 20% moenomycin C₃, prerably less than 15%;
- less than 20% moenomycin C₄, preferably less than 15%.

### COMPOUNDS AND/OR COMPOSITIONS FOR USE IN THE TREATMENT AND/OR PREVENTION OF ERE

The present invention further relates to a compound for use in the treatment and/or prevention of ERE, wherein the treatment and/or prevention comprises administering a daily dose of about 0.05 to about 100 mg of the compound. In a further embodiment, the treatment and/or prevention comprises admininstering a daily dose of about 0.1 to about 50 mg of the compound, in particular about 0.1 to about 30 mg, more in particular about 0.3 to about 20 mg. In a further embodiment, about 0.3 to about 20 mg, in particular about 0.3 to 12 mg, more in particular about 0.3 to 10 mg. In an even further embodiment, about 0.5 to about 6 mg.

In a further embodiment, the treatment and/or prevention comprises administering a daily dose of about 0.05 to about 50 mg of the compound according to the invention per kg bodyweight, in particular about 0.1 to about 25 mg/kg bodyweight, more in particular about 0.1 to about 15 mg/kg bodyweight. In a further embodiment, about 0.3 to about 10 mg/kg bodyweight, in particular about 0.3 to about 6 mg/kg bodyweight, more in particular about 0.3 to about 5 mg/kg bodyweight. In an even further embodiment, about 0.5 to about 3 mg/kg bodyweight.

In another embodiment, the treatment and/or prevention comprises administering the the compound or composition according to the invention for at least 3, in particular at least 5, more in particular at least 7 consecutive days. In a further embodiment, at least 10, preferably at least 14 consecutive days.

For the avoidance of doubt, if an embodiment of the invention refers to a specific amount of a compound of the invention, it refers to a specific amount of any of the compounds and preferred compounds mentioned herein, e.g. moenomycins or moenomycin A, or to a specific amount of a mixture of compounds and preferred compounds mentioned herein, e.g. a specific amount of bambermycin.

The invention further relates to a compound of use in the treatment and/or prevention of ERE, in particular the compounds for use of any of the classes (I) to (III), as defined above, wherein the compound is administered orally.

Compounds may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets.

Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

Compounds may also be administered via oral inhalation or insufflation by means of methods and formulations employed in the art for administration via this way. Compounds may be administered to the lungs in the form of a solution, a suspension or a dry powder, a solution being preferred. Any system developed for the delivery of solutions, suspensions or dry powders via oral inhalation or insufflation are suitable for the administration of compounds.

It is especially advantageous to formulate the aforementioned compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

Therefore, the present invention also provides a pharmaceutical composition for use in the prevention and/or treatment of ERE, the composition comprising a compound as defined herein and a pharmaceutically acceptable carrier. In one embodiment, the pharmaceutical composition is in a unit dosage form. In a further embodiment, the unit dosage form comprises the daily dosage to be administered, in particular the daily dosages as defined herein before. More in particular, the pharmaceutical composition is in a unit dosage form comprising 0.05 to 100 mg of the compounds as defined herein. Preferably, the pharmaceutical composition is in a unit dosage form comprising 0.05 to 100 mg of moenomycins, preferably 0.1 to 30 mg. In a further preferred embodiment, the pharmaceutical composition is in a unit dosage form comprising 0.05 to 100 mg of moenomycin A. In another embodiment, the pharmaceutical composition is in a unit dosage form comprising 0.05 to 100 mg of bambermycin, preferably 0.1 to 30 mg. As mentioned herein, the pharmaceutical composition is preferably an oral composition.The present invention further relates to a composition for use in the treatment and/or prevention of ERE, the composition comprising the compound as described above.

Preferably, the compound according to the invention is administered in combination with feed. Therefore, the present invention also provides rabbit feed comprising a compound for use as defined herein. The skilled person is well aware on how to convert daily dosage amounts to rabbit feed supplement concentrations and vice-versa. Therefore, the present invention also provides a feed composition comprising 1 to 300 ppm, in particular 1 to 200 ppm, more in particular 1 to 100 ppm of the compounds according to the present invention. In a further embodiment, the feed composition comprises about 1 to about 50 ppm of a compound as defined herein. In another embodiment, the feed composition comprises at least 3, in particular at least 5, more in particular at least 10 ppm of a compound as defined herein. In a preferred embodiment, the feed composition comprises between 3 and 100, in particular between 5 and 50 ppm, more in particular between 10 and 40 ppm of a compound as defined herein. In one embodiment, the feed composition comprises between 20 and 30 ppm, in particular about 24 ppm of the compounds as described herein.

The present disclosure further provides a method comprising mixing the compounds as described herein with rabbit feed (not claimed herein).

The following examples are merely illustrative of some non-limiting embodiments of the present invention.

### EXAMPLES

### MATERIALS

### a) Experimental set-up

Experiments were conducted to test the clinical effects of feed supplementation with 16ppm, 24ppm or 32ppm of bambermycin to prevent and treat Epizootic Rabbit Enteropathy (ERE). For all experiments described herein, the bambermycin used was the commercially available Flavomycin^{®}. Results were compared to a negative control group to estimate the appropriate dose.

Animals were weighed once at 35 days of age, grouped by litter origin and ranked by an increasing order of bodyweight. Afterwards, the animals were randomly allocated to any of the four test groups as detailed below. The animals were housed in 2 rooms containing each 40 experimental cages with 6 test rabbits and 2 inoculated rabbits per cage. Cage diameters are 46cm wide, 79cm deep and 29cm high. Artificial light was provided from 7:00 am to 5:00 pm and target temperature was maintained at 18°C.

The rabbits were divided in 4 groups of 160 animals each. Each group comprised 40 inoculated rabbits and 120 test rabbits. The 4 groups had following characteristics:

### 1) Infected + standard diet: negative control

Test rabbits were co-housed with inoculated rabbits on Day 1 of the experiment. All rabbits were fed ad libitum for 14 days with standard diet from the first day clinical symptoms of ERE were observed on at least 2 rabbits (inoculated or not). Dead weight, live weight, morbidity characteristics and feed consumption was measured at certain timepoints as detailed below.

### 2) Infected + diet supplemented with 16ppm bambermycin

Test rabbits were co-housed with inoculated rabbits on Day 1 of the experiment. All rabbits were fed ad libitum for 14 days with a diet supplemented with 16ppm bambermycin from the first day clinical symptoms of ERE were observed on at least 2 rabbits (inoculated or not). Dead weight, live weight, morbidity characteristics and feed consumption was measured at certain timepoints as detailed below.

### 3) Infected + diet supplemented with 24ppm bambermycin

Test rabbits were co-housed with inoculated rabbits on Day 1 of the experiment. All rabbits were fed ad libitum for 14 days with a diet supplemented with 24ppm bambermycin from the first day clinical symptoms of ERE were observed on at least 2 rabbits (inoculated or not). Dead weight, live weight, morbidity characteristics and feed consumption was measured at certain timepoints as detailed below.

### 4) Infected + diet supplemented with 32ppm bambermycin

Test rabbits were co-housed with inoculated rabbits on Day 1 of the experiment. All rabbits were fed ad libitum for 14 days with a diet supplemented with 32ppm bambermycin from the first day clinical symptoms of ERE were observed on at least 2 rabbits (inoculated or not). Dead weight, live weight, morbidity characteristics and feed consumption was measured at certain timepoints as detailed below.

### b) Verification of experimental set-up

Feed composition of the different experimental groups was analysed and presented in the table below:

| Feed | Humidity (g/100g) | Protein (g/100g) | Crude fibre (g/100g) | Starch (g/100g) |
|---|---|---|---|---|
| Standard (neg. control) | 9.9 | 14.8 | 18.9 | 12.4 |
| Supplemented with 16ppm bambermycin | 9.8 | 15.5 | 18.4 | 12.7 |
| Supplemented with 24ppm bambermycin | 9.9 | 15.2 | 17.8 | 12.2 |
| Supplemented with 32ppm bambermycin | 9.9 | 15.2 | 17.8 | 12.2 |
| *Analytical uncertainty* | *0.5* | *0.5* | 1.4 | 1.0 |

Characteristics of the feed composition of the different experimental groups were similar on humidity, protein, crude fibre and starch taking into account the given analytical uncertainty.

### METHODS

### a) Weight and feed intake measurements

All dead rabbits were weighed on the day of observation. All living rabbits (both test rabbits and inoculated rabbits) were weighed on:
- Day -1: day before allocation to treatment groups (i.e. 35 days of age)
- Day 8: i.e. 43 days of age
- Day 14: i.e. 49 days of age
- Day 21: i.e. 56 days of age
- Day 28: i.e. 63 days of age
- Day 35: i.e. 70 days of age

Rabbits were weighed also collectively (per cage) during the treatment period (including inoculated rabbits):
- The first day of the treatment period
- 3 times per week
- The last day of the treatment period

Out of the treatment period, feed consumption per cage was measured on the same days as the live weight measurements (Days 8, 14, 21, 28, 35). During the treatment period, feed intake was measured every day.

### b) Assessment of morbidity

To ensure only healthy animals were included in the study, each rabbit was subjected to veterinary examination when allocated to its treatment group (Day 1).

Both inoculated and test rabbits were observed for clinical signs when individual bodyweights were measured (Day 8, 14, 21, 28, 35). Clinical signs were observed by an experienced person blinded towards the treatment. Morbidity was also assessed at the beginning and at the end of the treatment.

Three clinical symptoms were registered, i.e. the absence or presence of diarrhoea, the absence or presence of tympany and the absence or presence of borborygmus. Any other clinical signs were recorded with a complete description.

### c) Assessment of mortality

Both inoculated and test rabbits that died during the study were recorded, weighed and subjected to visual observation to detect signs of diarrhoea (absence or presence), tympany (absence or presence) and borborygmus (absence or presence).

All animals that died in the course of the study, either naturally or by euthanasia, were daily sent to the laboratory in charge of post-mortem examination for necropsy. During weekends or on Fridays after 4:00 PM, dead rabbits were stored frozen and sent to the laboratory on the following Monday. If a dead rabbit was discovered after 4:00 PM during the week, it was stored at 4 degrees and sent to the laboratory on the following day.

Necropsy was performed on all deceased rabbits (both inoculated as well as test rabbits that died naturally or by euthanasia) by or under the supervision of the veterinarian of the laboratory in charge of post-mortem examination to determine the cause of mortality in view of the clinical signs and necropsy observations.

ERE-related signs are described below:
- Clinical signs (at least one of the signs described hereafter): rumbling noise, caecal impaction, diarrhoea, distended abdomen (tympany), excretion of mucus, decreased bodyweight gain compared to negative controls
- Necropsy signs (at least one of the signs described hereafter): dilatation of the stomach which is filled with liquid and gas, distension of the small intestine with large amount of liquid and possibly gas, caecal impaction (no signs of inflammation are usually observed), mucus in colon.

### d) End point observation

The end point is the combination of factors that are measured and observed. All animals meeting the criteria listed below are sent to the laboratory to be euthanized:
- An animal with a weight loss greater than 45 g/day
- Moribund animals (depressed, motionless, lateral decubitus)
- Glassy look or anorexia
- Having diarrhoea or having had diarrhoea
- Weak

### e) Statistical analyses

Summary statistics including mean, standard deviation, minimum and maximum value were produced stratified on treatment group for normally distributed outcome variables. Frequency tables stratified on group were created for nominal or ordinal data (mortality and necropsy scores, ERE prevalence categories). The statistical test was 2-sided with α=0,05, and 95% confidence intervals were used. The reference treatment group was the control group. Differences between treatment groups were described through the use of confidence intervals, and p-values up to 2 decimals and differences between groups are indicated through the use of superscripts in the statistical models. Univariate statistics and box plots of the distribution of the outcome variables were initially performed to determine the distributions to assess the appropriate statistical approach to use in modelling.

Survival analysis (Proportional Hazard models) and Kaplan Meier plots or statistics were used to describe mortality. Through survival analysis, not only the number of dead or prematurely removed rabbits can be assessed per group, but also the rate of mortality over the study period. The proportional hazard models included weight on Day 35, study room and inoculation status as covariates and a random effect of cage.

Clinical observations of ERE symptoms in live animals were evaluated on a daily basis per rabbit. All symptoms that are indicative as ERE were coded 1, whereas rabbits without ERE symptoms were coded as 0. The number of days that a rabbit showed signs of ERE were counted, and also calculated as % of days in the study.

The ERE days were not normally distributed, and although a mixed model is presented, the non-parametric models are more statistically correct. The rabbits were categorized into 4 ERE duration categories based on the % of days that they exhibited ERE symptoms. A cumulative logistic GEE model estimated the odds that a rabbit had increasing duration (belonged to a higher ERE duration category), while including a repeated measure on cage and testing the covariates study room, gender, inoculation status and day 35 weight. A GEE negative binomial model for total ERE symptoms was further used to evaluate the increasing number of days with ERE, where the dependent variable was the number of days that the rabbit presented ERE symptoms. The model included a repeated measure on cage, and the study room and inoculation status were retained in model regardless of significance. Other variables such as gender and weight on day 35 were tested for inclusion but omitted due to non-significance.

The post-mortem diagnosis were evaluate with stratified analysis, looking at the reason for exit of the rabbit (dead, censored, or inoculated and censored). Trends and differences were evaluated with Chi-square, Fisher Exact and Jonkheere-Terpstra test of trend. Furthermore a GEE Cumulative Logistic Model was used to look at the odds of having increasing ERE score at post-mortem with group affiliation and reason for exit as covariates, and a random effect of cage.

Data were analyzed by analysis of variance to detect any treatment effect. Statistical analyses were performed with R software (Version 0.98.1103).

### RESULTS

Treatment with bambermycin (Flavomycin^{®}) started on day 5 of the trial, the moment when 2 morbid rabbits were observed among the test animals. At this timepoint, the rabbits were 40 days of age. The treatment lasted 14 days and ended when the animals were 54 days of age (Day 19 of the trial).

Inoculated rabbits were euthanized on Day 21 of the trial.

### a) Mortality results

Figure 1 shows the ERE-related cumulative mortality rate (combining all rabbits, both inoculated and test rabbits). The mortality varied between 12.5% and 26.9% during the treatment period. In the control group, mortality was 26.9%, whereas for the groups receiving feed supplemented with 16ppm bambermycin, 24ppm bambermycin and 32ppm bambermycin the ERE-related mortality was 15.0%, 12.5% and 16.9%, respectively. Non-parametric test of trend (Jonkheere-Terpstra P-value <0.02) and Survival Analysis (Log Rank P-value <0.01) indicated that mortality was significantly different between groups. From Table 1 below, it can be concluded that the mortality in the group receiving feed supplemented with 24ppm bambermycin was significantly lower compared to control (upper confidence interval limit of 17.6% of the latter group was below the lower confidence interval limit of the control group, i.e. 20%).

**Table 1: Mortality percentage of animals in control and treatment groups**

| **Treatment** | **Total** # | **Dead** | **Censored** | **% Mortality** | **STD** |
|---|---|---|---|---|---|
| **Control** | 160 | 43 | 117 | 26,9% | 3,50% |
| **Bambermycin 16ppm** | 160 | 24 | 136 | 15,0% | 2,82% |
| **Bambermycin 24ppm** | 160 | 20 | 140 | 12,5% | 2,61% |
| **Bambermycin 32ppm** | 160 | 27 | 133 | 16,9% | 2,96% |

| | | | | | |
|---|---|---|---|---|---|
| Jonkheere-Terpstra test of trend P-value = 0,02 | | | | | |

If only the contact animals are considered, the same trend was observed. The mortality in the control group was 28.3%, whereas the mortality rate for the group receiving feed supplemented with 16ppm bambermycin, 24ppm bambermycin and 32ppm bambermycin was 15.8%, 12.5% and 18.3%, respectively. This data is shown in Table 2 below.

**Table 2: Mortality percentage of contacted animals in control and treatment groups**

| **Treatment** | **Total #** | **Dead** | **Censored** | **% Mortality** | **STD** |
|---|---|---|---|---|---|
| **Control** | 120 | 34 | 86 | 28,3% | 4,11% |
| **Bambermycin 16ppm** | 120 | 19 | 101 | 15,8% | 3,33% |
| **Bambermycin 24ppm** | 120 | 15 | 105 | 12,5% | 3,02% |
| **Bambermycin 32ppm** | 120 | 22 | 98 | 18,3% | 3,53% |

| | | | | | |
|---|---|---|---|---|---|
| Jonkheere-Terpstra test of trend P-value = 0,04 | | | | | |

If only the inoculated animals are considered, the same trend was observed. The mortality in the control group was 22.5%, whereas the mortality for the groups receiving feed supplemented with 16ppm bambermycin, 24ppm bambermycin and 32ppm bambermycin was 12.5% for each group.

A proportional hazard model indicated that the risk of dying in the bambermycin group was significantly lower (41-58% lower) than the control group (P-value < 0.01). The largest reduction in death risk was seen in the group receiving feed supplemented with 24ppm bambermycin, where the risk of dying was 58% lower compared to the control group. No further improvement was observed in the group receiving feed supplemented with 32ppm bambermycin. Table 3 below shows the results of the analysis using the proportional hazard model.

**Table 3: Proportional hazard model analysis of all animals**

| **Variable** | **Treatment** | **Estimate** | **S.E** | **Mortality** | **P-value** | **Pdiff** |
|---|---|---|---|---|---|---|
| | | | | **HR** | | |
| **A** | **Control** | 0,00 | 0,00 | 1,00 | ref. | a |
| **B** | **Bambermycin 16ppm** | -0,66 | 0,26 | 0,52 | <0,01 | b |
| **C** | **Bambermycin 24ppm** | -0,86 | 0,27 | 0,42 | <0,01 | b |
| **D** | **Bambermycin 32ppm** | -0,53 | 0,25 | 0,59 | 0,03 | b |
| Room | 1 | 0,24 | 0,19 | 1,28 | 0,20 | a |
| | 2 | 0,00 | 0,00 | 1,00 | ref. | a |
| BW Day 35 | one gram plus | -0,001 | 0,001 | 0,999 | 0,10 | |

| | | | | | | |
|---|---|---|---|---|---|---|
| ^{∗} random effect of cage | | | | | | |

When only the contact rabbit were considered, similar results were obtained. Mortality was significantly reduced in groups receiving feed supplemented with bambermycin compared to the control group. The mortality was reduced by 62% in the group receiving feed supplemented with 24ppm bambermycin. No further improvement was observed in the group receiving feed supplemented with 32ppm bambermycin. Table 4 below shows the results of the proportional hazard model analysis.

**Table 4: Proportional hazard model analysis of contacted animals**

| **Variable** | **Treatment** | **Estimate** | **S.E** | **Mortality** | **P-value** | **Pdiff** |
|---|---|---|---|---|---|---|
| | | | | **HR** | | |
| **A** | **Control** | 0,00 | 0,00 | 1,00 | ref. | a |
| **B** | **Bambermycin 16ppm** | -0,68 | 0,29 | 0,51 | 0,01 | b |
| **C** | **Bambermycin 24ppm** | -0,97 | 0,31 | 0,38 | <0,01 | b |
| **D** | **Bambermycin 32ppm** | -0,53 | 0,28 | 0,59 | 0,05 | b |
| Room | 1 | 0,32 | 0,21 | 1,38 | 0,13 | a |
| | 2 | 0,00 | 0,00 | 1,00 | ref. | a |
| BW Day 35 | one gram plus | -0,001 | 0,001 | 0,999 | 0,26 | |

| | | | | | | |
|---|---|---|---|---|---|---|
| ^{∗} random effect of cage | | | | | | |

Among the rabbits that died naturally, 74 showed ERE signs at necropsy. 30 showed slight signs of ERE and 6 had no signs of ERE.

Among the rabbits that were euthanized, 5 showed ERE signs at necropsy, 1 showed slight signs of ERE and 6 had no signs of ERE.

Among the rabbits that were inoculated and euthanized, 16 showed slight signs of ERE and 118 had no signs of ERE. They had probably recovered from the disease.

In total, 79 dead rabbits showed clear signs of ERE, 47 showed slight signs of ERE and 130 did not present signs of ERE. However these 130 rabbits comprised a large part of the rabbits that were inoculated and euthanized according to the planning in the protocol.

### b) Morbidity results

Figure 2 shows the evolution of the morbidity rate for the control group and three treatment groups. Based on the individual clinical observations, the highest incidence of animals demonstrating clinical signs of ERE was observed at 56 days of age. Morbidity was always highest in the control group, although not always significant. Morbidity was significantly different between groups on day 28 of the trial. At Day 8 and 21 of the trial, a tendency is observed. The causes of morbidity were mainly diarrhoea, bloating, borborygmus and their combinations.

Figure 3 shows for each group (either treatment or control) the number of rabbits for which a certain amount of days with ERE symptoms were registered. The groups receiving feed supplemented with 16ppm bambermycin, 24ppm bambermycin and 32ppm bambermycin (Least Square Mean (LSM) of 5.6% days) had significantly (P <0.01) less days with ERE compared to the control group (LSM of 9.1%). A summary of these data are shown in Table 5.

**Table 5: Percentage of days with ERE symptoms**

| **Group** | **Bambermycin treatment** | **% ERE** | **S.E** | **P-value** | **Pdiff** |
|---|---|---|---|---|---|
| | | **LSM** | | | |
| **A** | **Control** | 9,5% | 1,0% | ref. | a |
| **B** | **Bambermycin 16ppm** | 6,0% | 1,0% | <0,01 | b |
| **C** | **Bambermycin 24ppm** | 5,9% | 1,0% | <0,01 | b |
| **D** | **Bambermycin 32ppm** | 5,9% | 1,0% | <0,01 | b |
| | **Inoculated** | 6,7% | 1,2% | ref. | a |
| | **Contact** | 5,2% | 1,1% | 0,18 | a |

The incidence rate ratio (GEE Negative Binomial Model) of days with ERE in the groups that received feed supplemented with bambermycin are all significantly lower than the control group. The model indicates that the ERE incidence rate ratio (IRR) in the groups receiving feed supplemented with bambermycin are all significantly lower than the Control group. The model indicates that the IRR for the group receiving feed supplemented with 16ppm bambermycin was 45% lower, for the group receiving feed supplemented with 24ppm bambermycin was 41% lower and for the group receiving feed supplemented with 32ppm bambermycin was 37% lower. There was no significant difference between the 3 groups that received feed supplemented with bambermycin.

The odds (GEE Cumulative Logistic Regression Model) that a rabbit has increasing duration of ERE was highest in the Control group (reference) and significantly lower in all 3 treatment groups, with 56% lower in the group receiving feed supplemented with 16ppm bambermycin (P-value < 0,01), 43% lower in the group receiving feed supplemented with 24ppm bambermycin and 49% lower in the group receiving feed supplemented with 32ppm bambermycin.

### c) Live weight results

Table 6 shows a summary of the evolution of live bodyweight of the rabbits in all treatment groups and in the control group.

There was no significant difference in body weight between the four groups on Day 0.

On day 8 (age 43 days) the control rabbits (LSM of 1317g) and rabbits receiving feed supplemented with 16ppm bambermycin (LSM 1311g) were significantly lighter than rabbits receiving feed supplemented with 32ppm bambermycin (LSM 1338g). Rabbits receiving feed supplemented with 24ppm bambermycin (LSM 1325g) did not show a significantly different weight compared to the other groups.

On day 14 (age 49 days) the control rabbits (LSM 1497g) and rabbits receiving feed supplemented with 16ppm bambermycin (LSM 1516g) were significantly lighter than rabbits receiving feed supplemented with 24ppm bambermycin (LSM 1556g) and 32ppm bambermycin (LSM 1572g).

On day 21 (age 56 days) the control rabbits (LSM 1781g) were significantly lighter than rabbits receiving feed supplemented with 16ppm bambermycin (LSM 1841g) and 32ppm bambermycin (LSM 1854g). Rabbits receiving feed supplemented with 24ppm bambermycin (LSM 1830g) did not show a weight significantly different from rabbits in the other groups.

On day 28 (age 63 days) the control rabbits (LSM 2117g) were significantly lighter than rabbits receiving feed supplemented with 16ppm bambermycin (LSM 2199g) and 32ppm bambermycin (LSM 2198g). Rabbits receiving feed supplemented with 24ppm bambermycin (LSM 2175g) did not show significantly different weights compared to rabbits from other groups.

On day 35 (age 70 days) the control rabbits (LSM 2480g) were significantly lighter than rabbits receiving feed supplemented with 32ppm bambermycin (LSM 2553g). Rabbits receiving feed supplemented with 24ppm bambermycin (LSM 2532g) or 16ppm bambermycin (LSM 2539g) did not show weights significantly different from rabbits in other groups.

**Table 6: Live bodyweight evolution during the whole trial**

| **Bambermycin treatment** | **Live body weight (gram)** | | | | | |
|---|---|---|---|---|---|---|
| | **Day 35** | **Day 43** | **Day 49** | **Day 56** | **Day 63** | **Day 70** |
| **Control** | 949 | 1336 | 1505 | 1773 | 2116 | 2479 |
| **Bambermycin 16ppm** | 949 | 1330 | 1529 | 1830 | 2197 | 2537 |
| **Bambermycin 24ppm** | 949 | 1346 | 1572 | 1829 | 2183 | 2540 |
| **Bambermycin 32ppm** | 949 | 1356 | 1577 | 1841 | 2197 | 2552 |

### d) Average daily gain results

Table 7 shows the average daily gain (ADG) evolution during the whole trial. The ADG from Day 35 to 43 of age (study day 0 to 8) of the control rabbits (LSM 45,9g/day) and rabbits receiving feed supplemented with 16ppm bambermycin (LSM 45,4g/day) were significantly lower than the ADG of the rabbits receiving feed supplemented with 32ppm bambermycin (LSM 58,5g/day). Rabbits receiving feed supplemented with 24ppm bambermycin (LSM 46,9 gr/d) did not show an ADG that was significantly different from the other groups.

The ADG from Day 43 to 49 (study day 8 to 14) of the control rabbits (LSM 28,3g/day) and rabbits receiving feed supplemented with 16ppm bambermycin (LSM 32,4g/day) were significantly lower than the ADG of rabbits receiving feed supplemented with 24ppm bambermycin (LSM 38,1g/day) and 32ppm bambermycin (LSM 37,7g/day).

The ADG from Day 49 to 56 (study day 14 to 21) of the control rabbits (LSM 39,7g/day), rabbits receiving feed supplemented with 24ppm bambermycin (LSM 39,0g/day) and 32ppm bambermycin (LSM 39,9g/day) were significantly lower than the ADG of rabbits receiving feed supplemented with 16ppm bambermycin (LSM 45,6g/day).

There was no significant difference in ADG between control and treatment groups in the period from age Day 56 to 63 (study days 21 to 28). There was also no significant difference in ADG between treatment groups in the period from age Day 63 to 70 (study days 28 to 35).

**Table 7: Average daily gain evolution during the whole trial**

| **Bambermycin Treatment** | **Average daily gain (gram)** | | | | | |
|---|---|---|---|---|---|---|
| | **Day 35-43** | **Day 43-49** | **Day 49-56** | **Day 56-63** | **Day 63-70** | **Day 35-70** |
| **Control** | 48 | 27 | 37 | 51 | 52 | 44 |
| **Bambermycin 16ppm** | 48 | 32 | 43 | 52 | 49 | 45 |
| **Bambermycin 24ppm** | 49 | 37 | 36 | 50 | 51 | 45 |
| **Bambermycin 32ppm** | 51 | 37 | 37 | 52 | 51 | 46 |

### e) Feed intake results

The average daily feed intake (ADI) evolution for the control group and treatment groups is summarized in Table 8. There was no significant difference in average daily feed intake between groups during the period between 35 to 43 days of age (study days 0 to 8).

In the period between 43 and 49 days of age (study days 8 to 14), the ADI in control rabbits (LSM 77,9g/day) was significantly lower than the ADI of rabbits receiving feed supplemented with 24 ppm bambermycin (LSM 91,4g/day) and 32ppm bambermycin (LSM 93,3g/day) and marginally significantly lower than the ADI of rabbits receiving feed supplemented with 16ppm bambermycin (LSM 85,7g/day).

In the period between 49 to 56 days of age (study days 14 to 21), the ADI was significantly lower in the control group (LSM 113g/day) compared to the ADI of the group receiving feed supplemented with 16ppm bambermycin (LSM 125g/day), whereas the ADI in of rabbits receiving feed supplemented with 24ppm (LSM 117g/day) and 32ppm bambermycin (LSM 119g/day) was not significantly different from other groups.

There was no significant difference in ADI between the groups in the period between 56 to 63 days of age (study days 21 to 28) or 63 to 70 days of age (study days 28 to 35).

**Table 8: Average daily feed intake evolution during the whole trial**

| **Bambermycin Treatment** | **Average daily intake (gram)** | | | | | |
|---|---|---|---|---|---|---|
| | **Day 35-43** | **Day 43-49** | **Day 49-56** | **Day 56-63** | **Day 63-70** | **Day 35-70** |
| **Control** | 76 | 78 | 113 | 165 | 197 | 128 |
| **Bambermycin 16ppm** | 74 | 86 | 125 | 171 | 185 | 130 |
| **Bambermycin 24ppm** | 76 | 91 | 117 | 162 | 187 | 129 |
| **Bambermycin 32ppm** | 77 | 93 | 119 | 166 | 193 | 132 |

The ADI evolution for the different groups during the treatment period showed a significant effect or a tendency of effect of the treatment between 43 and 47 days of age. The ADI in the control group tended to be, or was significantly lower than the ADI of rabbits receiving feed supplemented with 32ppm bambermycin. At the age of 51 days, the ADI in the control group was significantly lower than the ADI of rabbits receiving feed supplemented with 16ppm bambermycin.

### e) Feed conversion ratio results

Table 9 summarizes the feed conversion ratio (FCR) evolution for the different groups. The FCR was significantly higher (P < 0,02) for the period between 35 and 43 days of age (study days 0 to 8) in the control group (LSM 1,6) compared to the group receiving feed supplemented with 32ppm bambermycin (LSM 1,52), whereas there were no significant differences regarding FCR between the groups receiving feed supplemented with 16ppm (LSM 1,56) and 24ppm bambermycin (LSM 1,54).

There was no significant difference in FCR between the groups for the periods between 43 to 49 days of age (study days 8 to 14), 49 to 56 days of age (study days 14 to 21), 56 to 63 days of age (study days 21 to 28) or 63 to 70 days of age (study days 28 to 35).

**Table 9: Feed conversion ratio evolution**

| **bambermycin treatment** | **Feed conversion ratio** | | | | | |
|---|---|---|---|---|---|---|
| | **Day 35-43** | **Day 43-49** | **Day 49-56** | **Day 56-63** | **Day 63-70** | **Day 35-70** |
| **Control** | 1,60 | 2,81 | 3,55 | 3,56 | 3,78 | 2,95 |
| **Bambermycin 16ppm** | 1,56 | 3,42 | 2,98 | 3,24 | 3,75 | 2,85 |
| **Bambermycin 24ppm** | 1,54 | 2,60 | 3,37 | 3,27 | 3,67 | 2,84 |
| **Bambermycin 32ppm** | 1,52 | 2,66 | 3,34 | 3,24 | 3,79 | 2,87 |

The overall FCR from 35 to 70 days of age (study days 0 to 35) was significantly lower between the control group (LSM 2,95) and the group receiving feed supplemented with 24ppm bambermycin. There was no significant difference in FCR between the group receiving feed supplemented 16ppm (LSM 2,85) or 32ppm (LSM 2,87) bambermycin compared to either the control group or the group receiving feed supplemented with 24ppm bambermycin.

### f) Bambermycin consumption

The consumption of bambermycin was calculated by dividing the consumption of bambermycin per rabbit by the average weight of the rabbit, i.e. the average of the weight of the rabbit at the end and the beginning of the treatment. The calculated bambermycin intake for the different groups is shown in Table 10 below:

**Table 10: Calculated bambermycin intake during the trial**

| **Bambermycin treatment** | **Bambermycin intake mg / kg bodyweight** |
|---|---|
| **Control** | 0 |
| **Bambermycin 12 ppm** | 12.1 |
| **Bambermycin 24 ppm** | 25.5 |
| **Bambermycin 32 ppm** | 39.5 |

### g) Conclusions

The objective of this study was to test the clinical efficacy of the compounds of the invention at three concentrations, i.e. 16, 24 and 32 ppm, in the diet during 14 days to reduce mortality in commercial rabbits suffering from Epizootic Rabbit Enteropathy (ERE) syndrome.

In this study, 2 rabbits per cage were inoculated with ERE. Feeds were compliant with expected values and results were consistent for low and mid-dose and somewhat lower for high dose.

Mortality was mainly related to ERE, validating the clinical model. The mortality was higher in the control group compared to the treated groups. Supplementing the feed with 24ppm of the compound of the invention provided the highest reduction in mortality. No further improvement was observed at a higher dose level.

The incidence rate ratio (GEE Negative Binomial Model) of days with ERE in the Flavomycin groups were all significantly lower than the control group.

Live weight was statistically different between groups; at 43days, 49 days, 56 days, 63 days and 70 days. Animals in the negative control group were lighter than animals in the group receiving feed supplemented with 32ppm bambermycin.

From 35 to 43 days of age, the ADG in the high dose groups was significantly higher than observed in the control group. From 43 to 49 days of age, the control group and group receiving feed supplemented with 16ppm bambermycin had a significantly lower ADG compared to rabbits receiving feed supplemented with 24ppm and 32 ppm bambermycin. From 49 to 56 days of age, the group receiving feed supplemented with 16 ppm bambermycin had the best ADG (significantly). After this date, there were no differences between groups. From 35 to 70 days of age, the group receiving feed supplemented with 32ppm bambermycin had a higher ADG compared to the control group.

On the whole trial, there was no difference between groups regarding feed consumption. The FCR was significantly better in group receiving feed supplemented with 24ppm bambermycin compared to the control group.

## Claims

1. A compound for use in the treatment and/or prevention of Epizootic Rabbit Enteropathy (ERE), wherein said compound is at least one of the compounds according to general formulae (I), (II), (III) or (IV), or the pharmaceutically acceptable salt, pharmaceutically acceptable solvate, or mixture thereof: 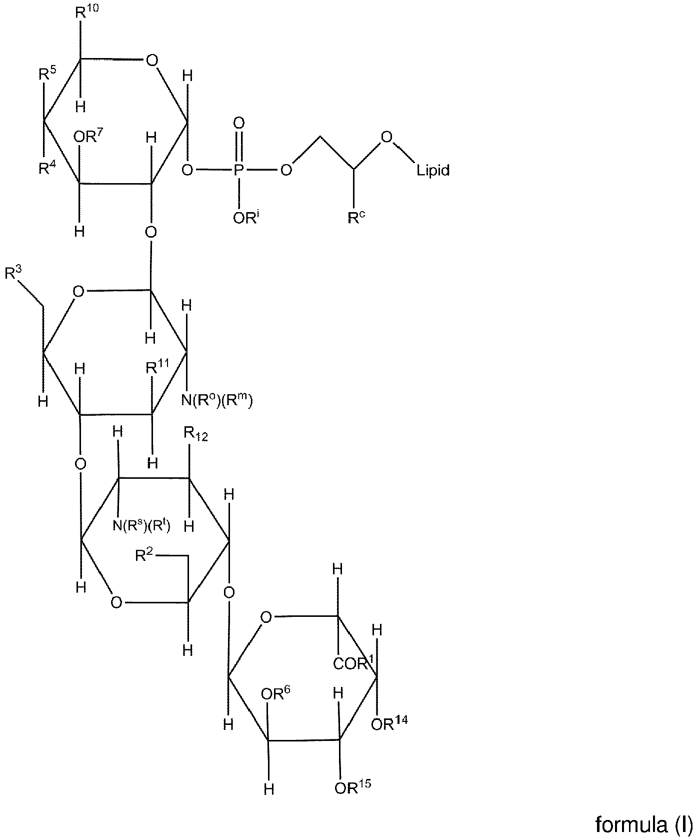 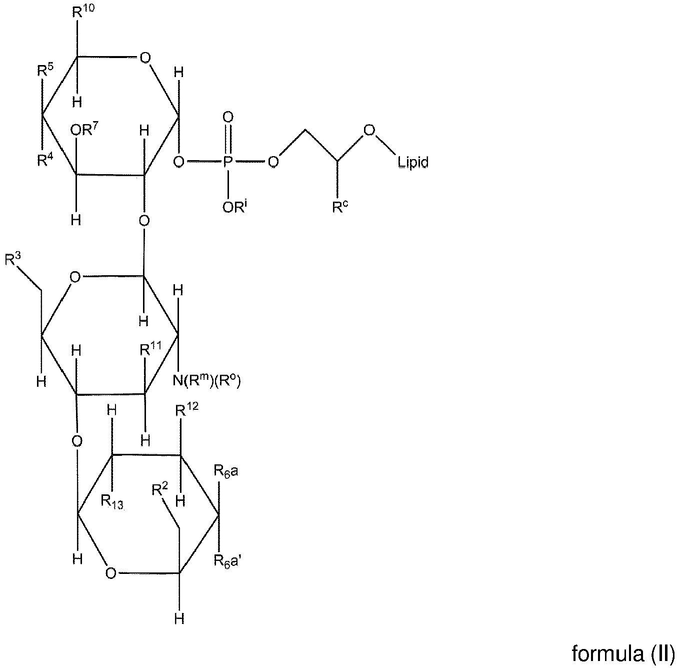 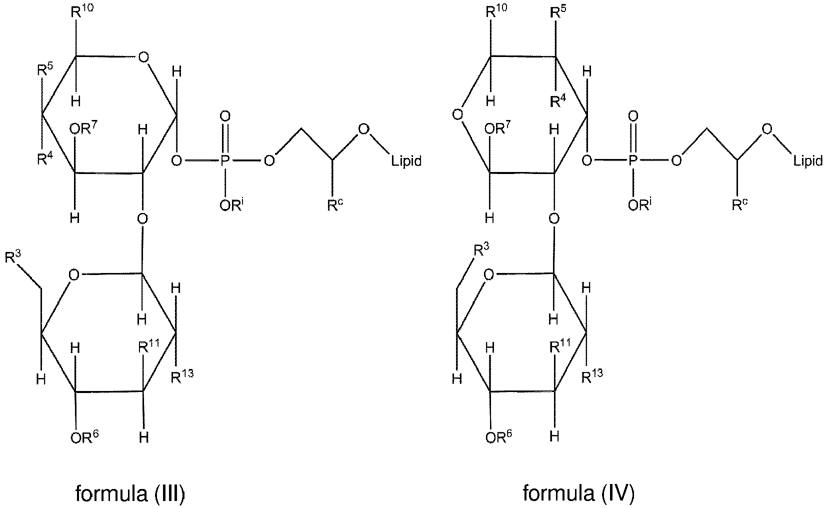 wherein
- each of R¹ is independently selected from -OH or -N(R^{1a})(R^{1b}), wherein each of R^{1a} and R^{1b}, independently from each other and at each occurrence, are selected from the group consisting of H, an amino protecting group, aryl, heteroaryl, aliphatic and a heteroaliphatic group,
- each of R², R⁴, R⁵, R¹¹ and R¹², independently from each other and at each occurrence, are selected from the group consisting of H, -OR^{z}, -N(R^{z})2, aryl, heteroaryl, aliphatic, and a heteroaliphatic group, wherein R^{z} is independently selected from the group consisting of H, an hydroxyl protecting group, an amino protecting group, aryl, heteroaryl, aliphatic, heteroaliphatic and a carbohydrate moiety,
- each of R³ is independently selected from the group consisting of H, -OH, -NH₂, -SH, OR^{w},-NH(R^{w}), -N(R^{w})₂, -SR^{w}, -O(C=O)R^{w}, -NH(C=O)R^{w}, -O(C=NH)R^{w}, -NH(C=NH)R^{w}, -S(C=NH))R^{w},
- NH(C=S)R^{w}, -S(C=O)R^{w}, -O(C=S)R^{w}, -S(=S)R^{w}, aryl, heteroaryl, aliphatic and a heteroaliphatic group, wherein R^{w} is selected from the group consisting of a carbohydrate moiety, aryl, heteroaryl, aliphatic, and heteroaliphatic group,
- each of R⁶, R¹⁴ and R¹⁵, independently from each other and at each occurrence are selected from the group consisting of H, hydroxyl protecting group, aryl, heteroaryl, aliphatic, heteroaliphatic group and carbohydrate moiety,
- each of R⁷ is independently selected from the group consisting of H, C(=O)N(R^{z'})₂, -C(=O)OR^{z'} , an hydroxyl protecting group, aryl, heteroaryl, aliphatic, heteroaliphatic group and carbohydrate moiety, wherein R^{z'} is independently selected from the group consisting of H, an hydroxyl protecting group, an amino protecting group, aryl, heteroaryl, aliphatic, and a heteroaliphatic group,
- each of R¹⁰ is independently selected from the group consisting of -C(=O)N(R^{l})(R^{p}), -C(=O)OR^{k}, and -CH₂OR^{k}, wherein R^{l} and R^{p} are independently from each other and at each occurrence selected from the group consisting of H, an amino protecting group, aryl, heteroaryl, aliphatic, heteroaliphatic group and a carbohydrate moiety, wherein R^{k} is independently selected from the group consisting of H, an hydroxyl protecting group, aryl, heteroaryl, aliphatic, heteroaliphatic group and a carbohydrate moiety,
- each of R¹³ is independently selected from the group consisting of -OH and -N(R^{o"})(R^{m"}) , wherein R^{o"} and R^{m"} independently from each other and at each occurrence, are selected from the group consisting of H, -C(=O)R^{h}, an amino protecting group, aryl, heteroaryl, aliphatic and heteroaliphatic group, wherein R^{h} is selected from the group consisting of a carbohydrate moiety, aryl, heteroaryl, aliphatic and heteroaliphatic group,
- each of (R°) and (R^{m}), independently from each other and at each occurrence, are selected from the group consisting of H, -C(=O)R^{w'}, an amino protecting group, aryl, heteroaryl, aliphatic and heteroaliphatic group, wherein R^{w'} is selected from the group consisting of a carbohydrate moiety, aryl, heteroaryl, aliphatic and heteroaliphatic group;
- each of (R^{s}) and (R^{t}), independently from each other and at each occurrence are selected from the group consisting of H, -C(=O)R^{w"}, an amino protecting group, aryl, heteroaryl, aliphatic and heteroaliphatic group, wherein R^{w"} is selected from the group consisting of a carbohydrate moiety, aryl, heteroaryl, aliphatic and heteroaliphatic group;
- each of R^{6a} and R^{6a'}, independently from each other and at each occurrence are selected from H or -OH, preferably R^{6a} is H and R^{6a'} is -OH;
- each of R^{c} is independently selected from the group consisting of H, halogen, heteroaryl, -OR^{q},
- N(R^{q})₂, -SR^{q}, NO₂, -NC, -CN, -N₃, -N(R^{q})=NR^{q}, -CHO, -C(=O)R^{q}, -C(=S)R^{q}, C(=NR^{q})R^{q},-C(=O)OR^{q}, -C(=NR^{q})OR^{q}, -C(=NR^{q})N(R^{q})₂, -C(=O)N(R^{q})₂, -C(=S)OR^{q}, -C(=O)SR^{q}, -C(=S)SR^{q},-P(=O)(OR^{q})₂, -S(=O)(OR^{q}), -S(=0)₂(OR^{q}), -P(=O)N(R^{q})₂, -P(=O)₂N(R^{q})₂, -C(=0)NR'S(=O)₂R^{q},-S(=O)N(R^{q})₂ and -S(=O)₂N(R^{q})₂, wherein each of R^{q} is independently selected from the group consisting of H, aliphatic, heteroaliphatic, aryl, heteroaryl, and an hydroxyl protecting group,
- each of Rⁱ is independently selected from the group consisting of H, an hydroxyl protecting group, aliphatic, heteroaliphatic, aryl, and heteroaryl,
- each Lipid is independently selected from H or a C₁₋₃₀ aliphatic moiety, wherein 0 to 10 methylene units are optionally replaced with -O-, -NR^{x}-, -S-, -C(=O)-, -C(=NR^{x})-, -S(=O)-,-S(=O)₂-, -N=N-, -C=N-, -C(R^{y})=C(R^{y'})-, -N-O-, an arylene, or an heteroarylene moiety, wherein each of R^{x} is independently selected from the group consisting of H, aliphatic, heteroaliphatic, aryl, heteroaryl, or an amino protecting group, and wherein each of R^{y} and R^{y'}, independently from each other and at each occurrence are selected from the group consisting of H, aliphatic, heteroaliphatic, aryl and heteroaryl group.

2. The compound for use according to claim 1, wherein said compound is at least one of the compounds according to general formula (la), or the pharmaceutically acceptable salt, pharmaceutically acceptable solvate, or mixture thereof, 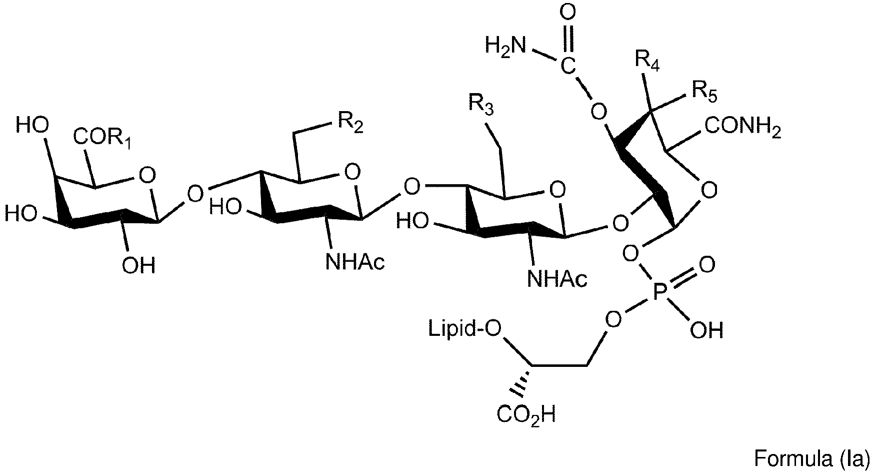 wherein
- each of R¹ is independently selected from OH, NH₂, or NH(R^{1b}), wherein R^{1b} is a C₁₋₁₀ cycloalkyl group or C₁₋₁₀ cycloalkenyl which is optionally substituted with an halide, hydroxyl or alkoxy, more preferably R¹ is -NH(R^{1b}), wherein R^{1b} is of following formula: 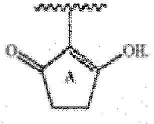
- each of R³ is independently selected from the group consisting of H, -OH and -OR^{w}, wherein R^{w} is D-glucose;
- each of R² and R⁴, independently from each other and at each occurrence are selected from H or -OH;
- each of R⁵ is independently selected from the group consisting of H, -OH and -CH₃;
- the Lipid is chosen among following Formula L₁ or Formula L₂:

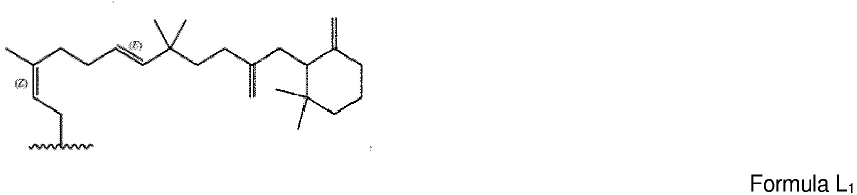

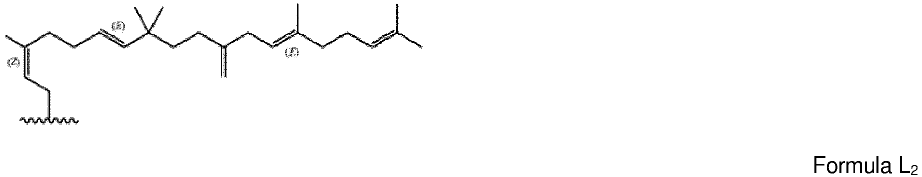

3. The compound for use according to claim 1 or claim 2, wherein said compound is at least one of the compounds according to general formula (Ib) or the pharmaceutically acceptable salt, pharmaceutically acceptable solvate, or mixture thereof,

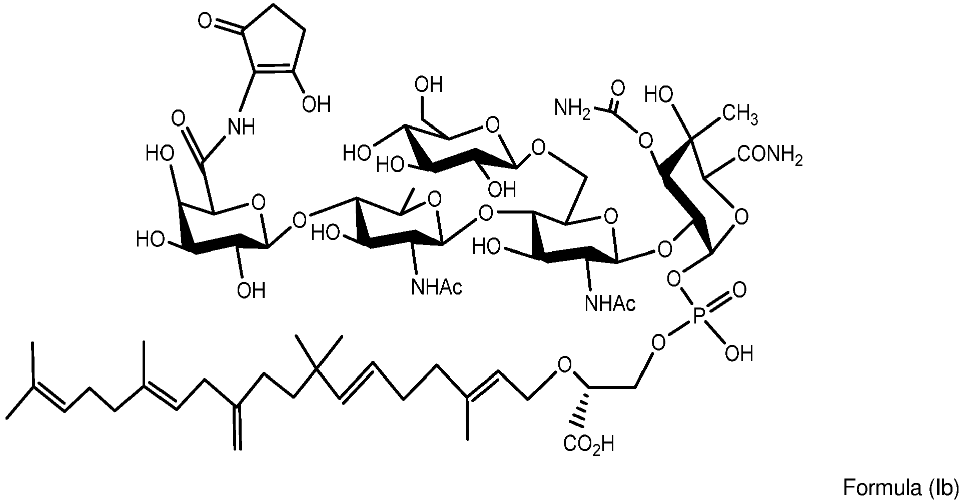

4. The compound for use according to claim 1, wherein said compound is at least one of the compounds according to formula (IIa) or formula (IIIa), or the pharmaceutically acceptable salt, pharmaceutically acceptable solvate, or mixture thereof: 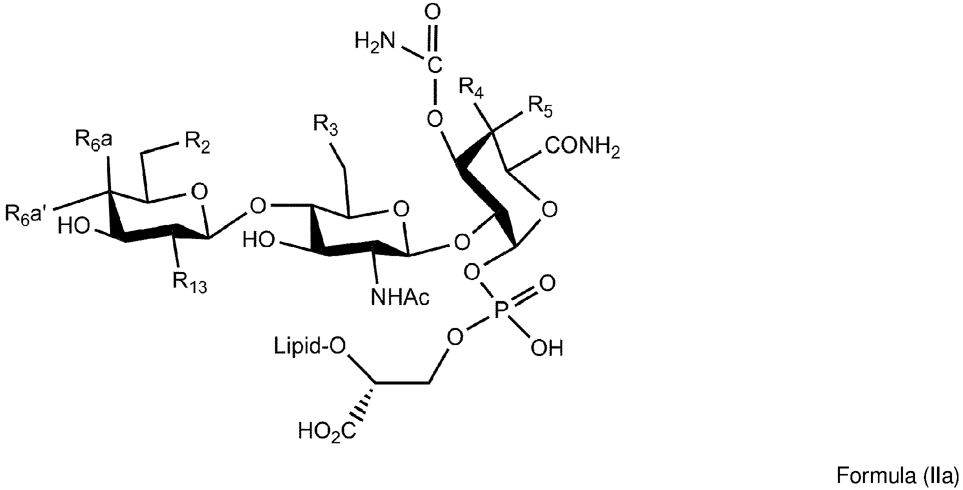 wherein
- R³ is H or -OH;
- each of R² and R⁴, independently from each other and at each occurrence, are selected from H or-OH;
- each of R^{6a} and R^{6a}, independently from each other and at each occurrence, are selected from H or -OH, preferably R^{6a} is H and R^{6a'} is -OH;
- R⁵ is H, -OH or CH₃;
- R¹³ is -OH or -NHC(=O)CH₃
- the Lipid is chosen among following Formula L₃, Formula L₄ or Formula L₅: 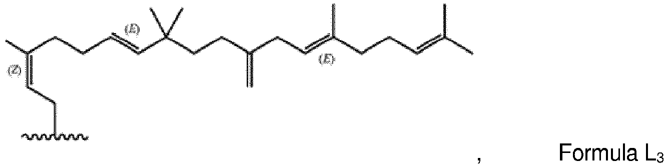 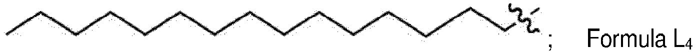 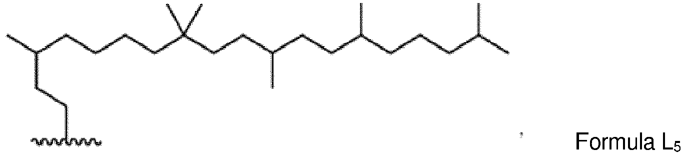
or 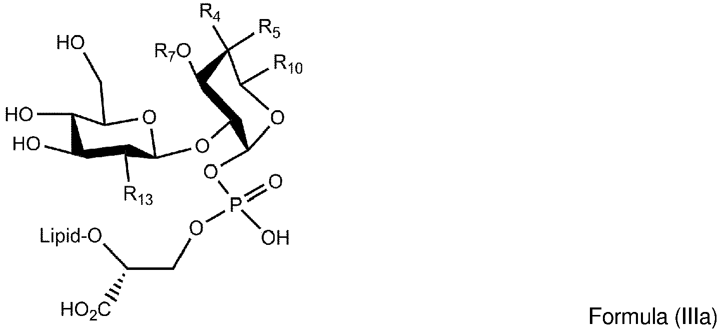 wherein
- each of R⁴ and R⁵, independently from each other and at each occurrence are selected from the group consisting of H, -CH₃ and -OH;
- R₇ is independently selected from H or -C(=O)NH₂, preferably, R₇ is C(=O)NH₂;
- R¹⁰ is independently selected from -C(=O)NH₂ or -C(=O)OH, preferably, R¹⁰ is C(=O)NH₂.;
- R¹³ is independently selected from the group consisting of -OH or -NHC(=O)CH₃, preferably R¹³ is -NHC(=O)CH₃
- the Lipid is chosen among following Formula L₆, Formula L₇ or Formula L₈:

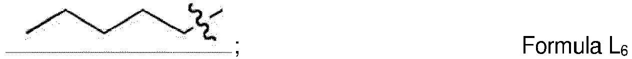

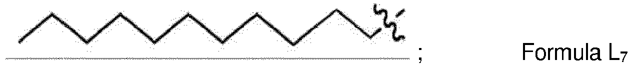

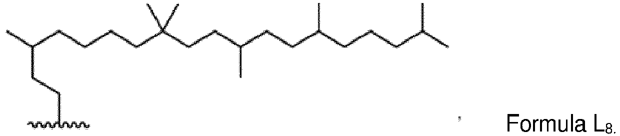

5. The compound for use according to any one of claims 1 to 4, wherein the treatment and/or prevention comprises administering a daily dose of 0.5 to 100 mg of the compound.

6. The compound for use according to any one of the previous claims, wherein the treatment and/or prevention comprises administering a daily dose of 0.5 to 3 mg/kg bodyweight.

7. The compound for use according to any one of claims 1 to 6, wherein the compound is administered orally.

8. The compound for use according to any one of the previous claims, wherein the compound is administered at least 10, preferably at least 14, consecutive days.

9. A pharmaceutical composition for use in the treatment and/or prevention of ERE, the composition comprising a compound as defined in any one of claims 1 to 4 and one or more pharmaceutically acceptable carriers.

10. The pharmaceutical composition for use according to claim 9, wherein the composition comprises 0.05 to 100 mg of the compound as defined in any one of claim 1 to 4.

11. A rabbit feed composition comprising a compound as defined in any one of claims 1 to 4 for use in the treatment and/or prevention of ERE.

12. The rabbit feed composition for use of claim 11, wherein the composition comprises about 1 to about 100 ppm of the compound as defined in any one of claims 1 to 4.

13. The rabbit feed composition for use of claim 11 or 12, wherein the treatment and/or prevention comprises orally administering the compound as defined in any one of claims 1 to 4 in the rabbit feed composition in a daily dose of 0.5 to 3 mg/kg bodyweight for at least 10, preferably at least 14, consecutive days.

14. The pharmaceutical or rabbit feed composition for use according to any one of claims 9 to 13, wherein the composition comprises bambermycin.

## Patentansprüche

1. Eine Verbindung zur Verwendung bei der Behandlung und/oder Vorbeugung von Epizootischer Enteropathie beim Kaninchen (Epizootic Rabbit Enteropathy - ERE), wobei die Verbindung zumindest eine der Verbindungen gemäß der allgemeinen Formel (I), (II), (III) oder (IV), oder das pharmazeutisch akzeptable Salz, pharmazeutisch akzeptable Solvat, oder eine Mischung davon ist: 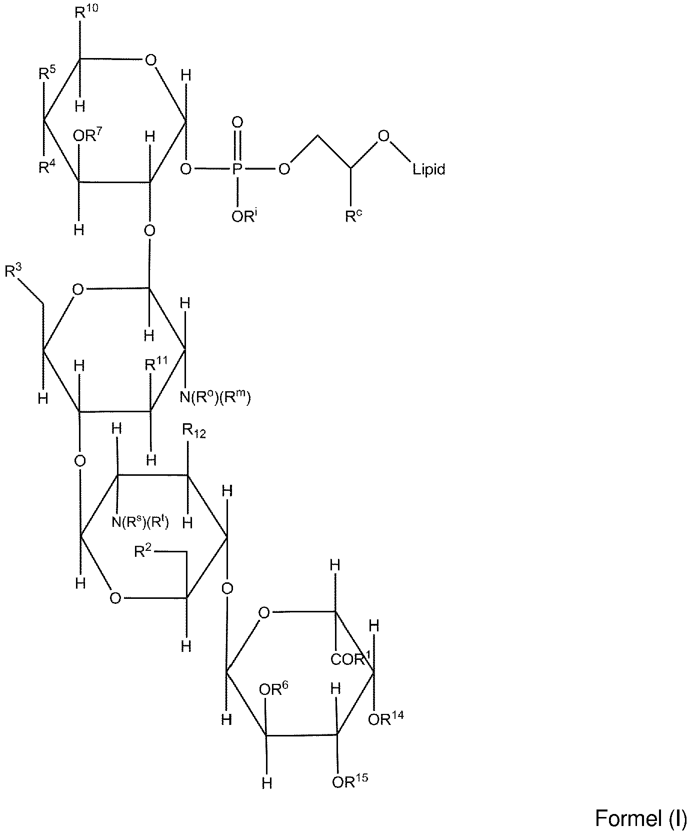 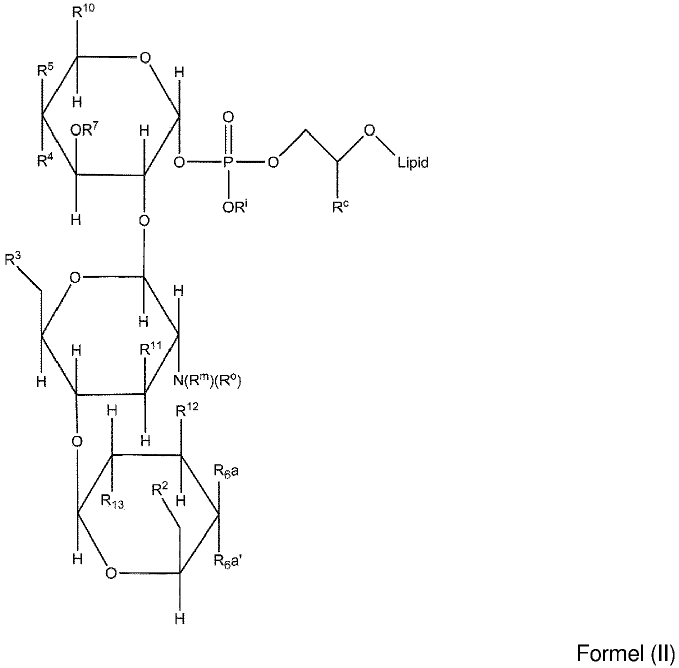 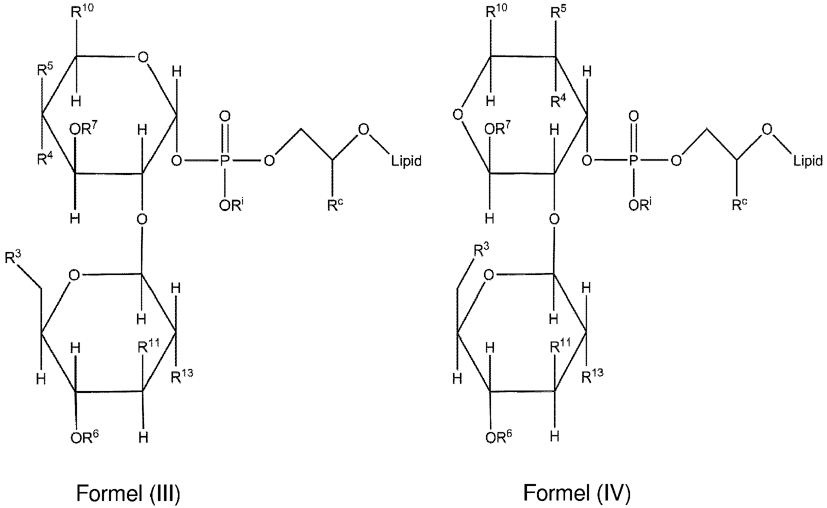 wobei
- jedes R¹ unabhängig ausgewählt ist aus -OH oder -N(R^{1a})(R^{1b}), wobei jedes R^{1a} und R^{1b}, unabhängig voneinander und bei jedem Vorkommen, ausgewählt ist aus der Gruppe bestehend aus H, einer Aminoschutzgruppe, Aryl-, Heteroaryl-, aliphatischen und einer heteroaliphatischen Gruppe,
- jedes R², R⁴, R⁵, R¹¹ und R¹², unabhängig voneinander und bei jedem Vorkommen, ausgewählt ist aus der Gruppe bestehend aus H, -OR^{z}, -N(R^{z})₂, Aryl-, Heteroaryl-, aliphatischer, und einer heteroaliphatischen Gruppe, wobei R^{z} unabhängig ausgewählt ist aus der Gruppe bestehend aus H, einer Hydroxylschutzgruppe, einer Aminoschutzgruppe, Aryl-, Heteroaryl-, aliphatischem, heteroaliphatischem und einem Kohlenhydratanteil,
- jedes R³ unabhängig ausgewählt ist aus der Gruppe bestehend aus H, -OH, -NH₂, -SH, OR^{w},
- NH(R^{w}), -N(R^{w})₂, -SR^{w}, -O(C=O)R^{w}, -NH(C=O)R^{w}, -O(C=NH)R^{w}, -NH(C=NH)R^{w}, -S(C=NH))R^{w},
- NH(C=S)R^{w}, -S(C=O)R^{w}, -O(C=S)R^{w}, -S(=S)R^{w}, Aryl-, Heteroaryl-, aliphatischer und einer heteroaliphatischen Gruppe, wobei R^{w} ausgewählt ist aus der Gruppe bestehend aus einem Kohlenhydratanteil, einer Aryl-, Heteroaryl-, aliphatischen, und heteroaliphatischen Gruppe,
- jedes R⁶, R¹⁴ und R¹⁵, unabhängig voneinander und bei jedem Vorkommen ausgewählt ist aus der Gruppe bestehend aus H, Hydroxylschutzgruppe, Aryl-, Heteroaryl-, aliphatischer, heteroaliphatischer Gruppe und Kohlenhydratanteil,
- jedes R⁷ unabhängig ausgewählt ist aus der Gruppe bestehend aus H, C(=O)N(R^{z'})₂,-C(=O)OR^{z'}, einer Hydroxylschutzgruppe, Aryl-, Heteroaryl-, aliphatischen, heteroaliphatischen Gruppe und einem Kohlenhydratanteil, wobei R^{z'} unabhängig ausgewählt ist aus der Gruppe bestehend aus H, einer Hydroxylschutzgruppe, einer Aminoschutzgruppe, Aryl-, Heteroaryl-, aliphatischen und einer heteroaliphatischen Gruppe,
- jedes R¹⁰ unabhängig ausgewählt ist aus der Gruppe bestehend aus -C(=O)N(R^{l})(R^{p}),-C(=O)OR^{k}, und -CH₂OR^{k}, wobei R^{l} und R^{p} unabhängig voneinander und bei jedem Vorkommen ausgewählt sind aus der Gruppe bestehend aus H, einer Aminoschutzgruppe, Aryl-, Heteroaryl-, aliphatischen, heteroaliphatischen Gruppe und einem Kohlenhydratanteil, wobei R^{k} unabhängig ausgewählt ist aus der Gruppe bestehend aus H, einer Hydroxylschutzgruppe, Aryl-, Heteroaryl-, aliphatischen, heteroaliphatischen Gruppe und einem Kohlenhydratanteil,
- jedes R¹³ unabhängig ausgewählt ist aus der Gruppe bestehend aus -OH und -N(R^{o"})(R^{m"}), wobei R^{o"} und R^{m"} unabhängig voneinander und bei jedem Vorkommen ausgewählt sind aus der Gruppe bestehend aus H, -C(=O)R^{h}, einer Aminoschutzgruppe, Aryl-, Heteroaryl-, aliphatischen und heteroaliphatischen Gruppe, wobei R^{h} ausgewählt ist aus der Gruppe bestehend aus einem Kohlenhydratanteil, einer Aryl-, Heteroaryl-, aliphatischen und heteroaliphatischen Gruppe,
- jedes (R°) und (R^{m}), unabhängig voneinander und bei jedem Vorkommen, ausgewählt ist aus der Gruppe bestehend aus H, -C(=O)R^{w'}, einer Aminoschutzgruppe, Aryl-, Heteroaryl-, aliphatischen und heteroaliphatischen Gruppe, wobei R^{w'} ausgewählt ist aus der Gruppe bestehend aus einem Kohlenhydratanteil, einer Aryl-, Heteroaryl-, aliphatischen und heteroaliphatischen Gruppe;
- jedes (R^{s}) und (R^{t}), unabhängig voneinander und bei jedem Vorkommen, ausgewählt ist aus der Gruppe bestehend aus H, -C(=O)R^{w"}, einer Aminoschutzgruppe, Aryl-, Heteroaryl-, aliphatischen und heteroaliphatischen Gruppe, wobei R^{w"} ausgewählt ist aus der Gruppe bestehend aus einem Kohlenhydratanteil, einer Aryl-, Heteroaryl-, aliphatischen und heteroaliphatischen Gruppe;
- jedes R^{6a} und R^{6a'}, unabhängig voneinander und bei jedem Vorkommen, ausgewählt ist aus H oder -OH, bevorzugt ist R^{6a} H und ist R^{6a'} -OH;
- jedes R^{c} unabhängig ausgewählt ist aus der Gruppe bestehend aus H, Halogen, Heteroaryl,-OR^{q}, -N(R^{q})₂, -SR^{q}, NO₂, -NC, -CN, -N₃, -N(R^{q})=NR^{q}, -CHO, -C(=O)R^{q}, -C(=S)R^{q}, C(=NR^{q})R^{q},-C(=O)OR^{q}, -C(=NR^{q})OR^{q}, -C(=NR^{q})N(R^{q})₂, -C(=O)N(R^{q})₂, -C(=S)OR^{q}, -C(=O)SR^{q}, -C(=S)SR^{q},-P(=O)(OR^{q})₂, -S(=O)(OR^{q}), -S(=0)₂(OR^{q}), -P(=O)N(R^{q})₂, -P(=O)₂N(R^{q})₂, -C(=0)NR'S(=O)₂R^{q},-S(=O)N(R^{q})₂ und -S(=O)₂N(R^{q})₂, wobei jedes R^{q} unabhängig ausgewählt ist aus der Gruppe bestehend aus H, aliphatischer, heteroaliphatischer, Aryl-, Heteroaryl-, und einer Hydroxylschutzgruppe,
- jedes Rⁱ unabhängig ausgewählt ist aus der Gruppe bestehend aus H, einer Hydroxylschutzgruppe, aliphatisch, heteroaliphatisch, Aryl, und Heteroaryl,
- jedes Lipid unabhängig ausgewählt ist aus H oder einem C₁₋₃₀ aliphatischen Anteil, wobei 0 bis 10 Methyleneinheiten optional ersetzt sind durch -O-, -NR^{x}-, -S-, -C(=O)-, -C(=NR^{x})-, -S(=O)-,-S(=O)₂-, -N=N-, -C=N-, -C(R^{y})=C(R^{y})-, -N-O-, einen Arylen-, oder einen Heteroarylenanteil, wobei jedes R^{x} unabhängig ausgewählt ist aus der Gruppe bestehend aus H, aliphatischer, heteroaliphatischer, Aryl-, Heteroaryl-, oder einer Aminoschutzgruppe, und wobei jedes R^{y} und R^{y'}, unabhängig voneinander und bei jedem Vorkommen, ausgewählt ist aus der Gruppe bestehend aus H, aliphatischer, heteroaliphatischer, Aryl- und Heteroarylgruppe.

2. Die Verbindung zur Verwendung nach Anspruch 1, wobei die Verbindung zumindest eine der Verbindungen gemäß der allgemeinen Formel (la), oder das pharmazeutisch akzeptable Salz, pharmazeutisch akzeptable Solvat, oder eine Mischung davon ist, 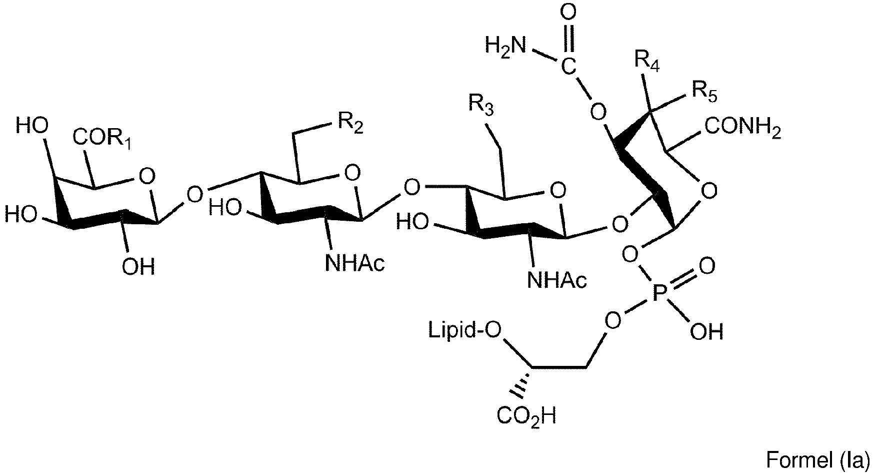 wobei
- jedes R¹ unabhängig ausgewählt ist aus OH, NH2, oder NH(R^{1b}), wobei R^{1b} eine C₁₋₁₀-Zykloalkylgruppe oder C₁₋₁₀-Zykloalkenyl ist, welches optional substituiert ist durch ein Halid, Hydroxyl oder Alkoxy, noch besser ist R¹-NH(R^{1b}), wobei R^{1b} folgende Formel aufweist: 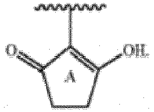
- jedes R³ unabhängig ausgewählt ist aus der Gruppe bestehend aus H, -OH und -OR^{w}, wobei R^{w} D-Glucose ist;
- jedes R² und R⁴, unabhängig voneinander und bei jedem Vorkommen, ausgewählt ist aus H oder -OH;
- jedes R⁵ unabhängig ausgewählt ist aus der Gruppe bestehend aus H, -OH und -CH₃;
- das Lipid gewählt wird aus der folgenden Formel L₁ oder Formel L₂:

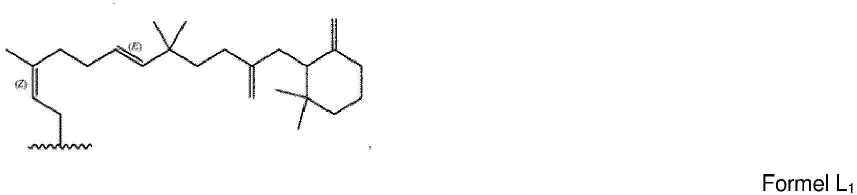

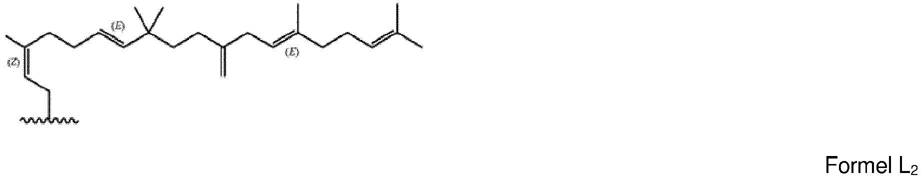

3. Die Verbindung zur Verwendung nach Anspruch 1 oder Anspruch 2, wobei die Verbindung zumindest eine der Verbindungen gemäß der allgemeinen Formel (Ib) oder das pharmazeutisch akzeptable Salz, pharmazeutisch akzeptable Solvat, oder eine Mischung davon ist,

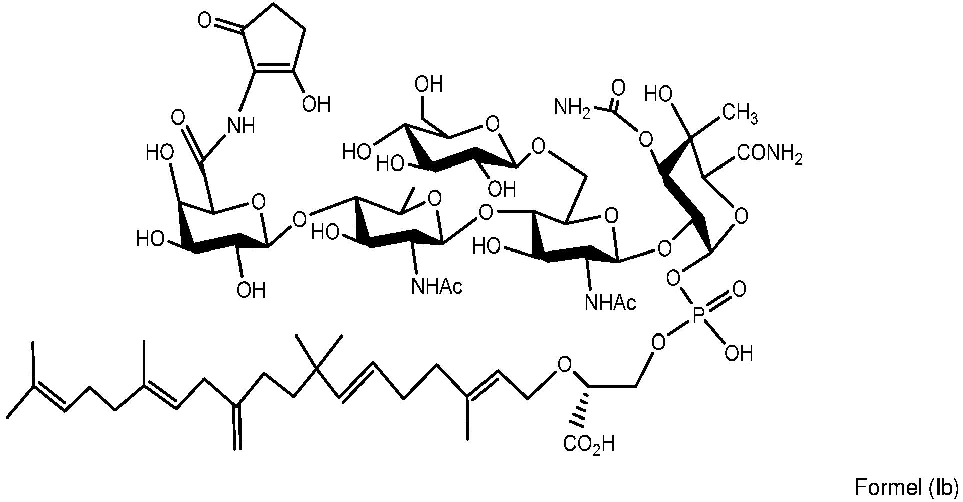

4. Die Verbindung zur Verwendung nach Anspruch 1, wobei die Verbindung zumindest eine der Verbindungen gemäß der Formel (IIa) oder Formel (lila), oder das pharmazeutisch akzeptable Salz, pharmazeutisch akzeptable Solvat, oder eine Mischung davon ist: 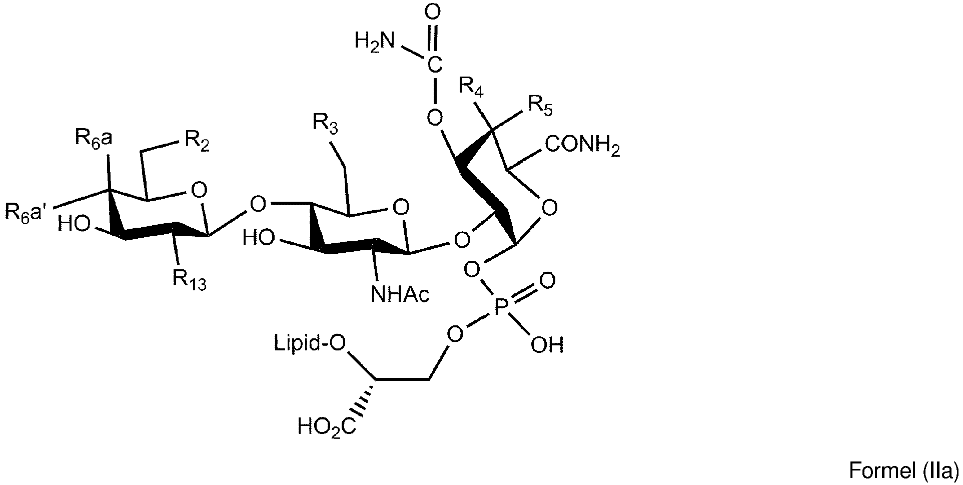 wobei
- R³ H oder -OH ist;
- jedes R² und R⁴, unabhängig voneinander und bei jedem Vorkommen, ausgewählt ist aus H oder -OH;
- jedes R^{6a} und R^{6a'}, unabhängig voneinander und bei jedem Vorkommen, ausgewählt ist aus H oder -OH, bevorzugt ist R^{6a} H und ist R^{6a'} -OH;
- R⁵ H, -OH oder CH₃ ist;
- R¹³ -OH oder -NHC(=O)CH₃ ist;
- das Lipid gewählt wird aus der folgenden Formel L₃, Formel L₄ oder Formel L₅: 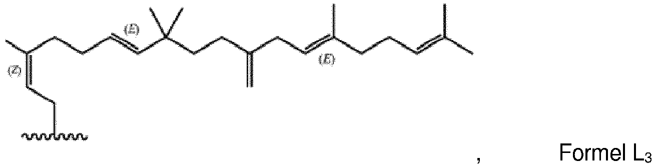 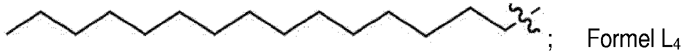 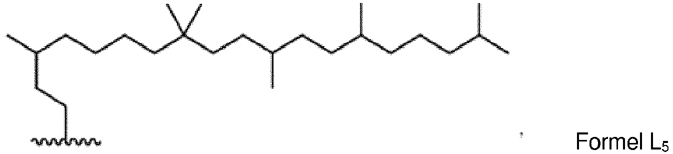
oder 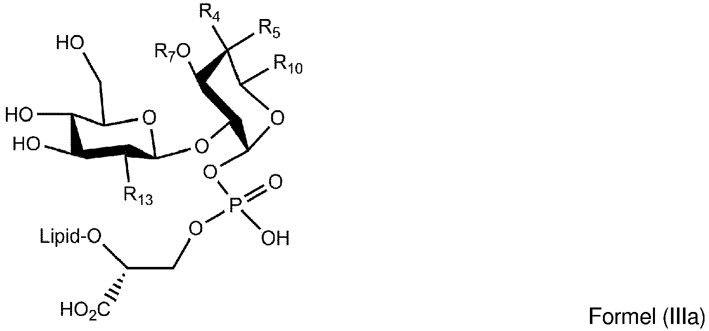 wobei
- jedes R⁴ und R⁵, unabhängig voneinander und bei jedem Vorkommen, ausgewählt ist aus der Gruppe bestehend aus H, -CH₃ und -OH;
- R₇ unabhängig ausgewählt ist aus H oder -C(=O)NH₂, bevorzugt ist R₇ C(=O)NH₂;
- R¹⁰ unabhängig ausgewählt ist aus -C(=O)NH₂ oder -C(=O)OH, bevorzugt ist R¹⁰ C(=O)NH_{2.};
- R¹³ unabhängig ausgewählt ist aus der Gruppe bestehend aus -OH oder -NHC(=O)CH₃, bevorzugt ist R¹³-NHC(=O)CH₃
- das Lipid gewählt wird aus der folgenden Formel L₆, Formel L₇ oder Formel L₈:

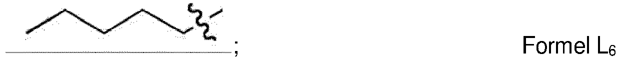

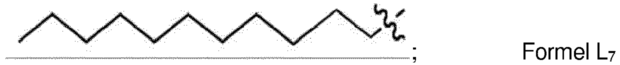

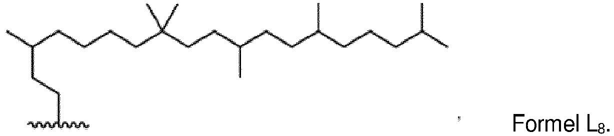

5. Die Verbindung zur Verwendung nach irgendeinem der Ansprüche 1 bis 4, wobei die Behandlung und/oder Vorbeugung das Verabreichen einer täglichen Dosis von 0,5 bis 100 mg der Verbindung umfasst.

6. Die Verbindung zur Verwendung nach irgendeinem der vorigen Ansprüche, wobei die Behandlung und/oder Vorbeugung das Verabreichen einer täglichen Dosis von 0,5 bis 3 mg/kg Körpergewicht umfasst.

7. Die Verbindung zur Verwendung nach irgendeinem der Ansprüche 1 bis 6, wobei die Verbindung oral verabreicht wird.

8. Die Verbindung zur Verwendung nach irgendeinem der vorigen Ansprüche, wobei die Verbindung mindestens 10, bevorzugt mindestens 14 Tage hintereinander verabreicht wird.

9. Eine pharmazeutische Zusammensetzung zur Verwendung bei der Behandlung und/oder Vorbeugung von ERE, wobei die Zusammensetzung eine Verbindung wie definiert in irgendeinem der Ansprüche 1 bis 4 und einen oder mehrere pharmazeutisch akzeptable Träger umfasst.

10. Die pharmazeutische Zusammensetzung zur Verwendung nach Anspruch 9, wobei die Zusammensetzung 0,05 bis 100 mg der Verbindung wie definiert in irgendeinem der Ansprüche 1 bis 4 umfasst.

11. Eine Kaninchenfutterzusammensetzung, welche eine Verbindung wie definiert in irgendeinem der Ansprüche 1 bis 4 zur Verwendung bei der Behandlung und/oder Vorbeugung von ERE umfasst.

12. Die Kaninchenfutterzusammensetzung zur Verwendung aus Anspruch 11, wobei die Zusammensetzung etwa 1 bis etwa 100 ppm der Verbindung wie definiert in irgendeinem der Ansprüche 1 bis 4 umfasst.

13. Die Kaninchenfutterzusammensetzung zur Verwendung aus Anspruch 11 oder 12, wobei die Behandlung und/oder Vorbeugung das orale Verabreichen der Verbindung wie definiert in irgendeinem der Ansprüche 1 bis 4 in der Kaninchenfutterzusammensetzung in einer täglichen Dosis von 0,5 bis 3 mg/kg Körpergewicht mindestens 10, bevorzugt mindestens 14 Tage hintereinander umfasst.

14. Die pharmazeutische oder Kaninchenfutterzusammensetzung zur Verwendung nach irgendeinem der Ansprüche 9 bis 13, wobei die Zusammensetzung Bambermycin umfasst.

## Revendications

1. Composé pour utilisation dans le traitement et/ou la prévention de l'entéropathie épizootique du lapin (EEL), dans lequel ledit composé est au moins l'un des composés selon les formules générales (I), (II), (III) ou (IV), ou le sel pharmaceutiquement acceptable, le solvate pharmaceutiquement acceptable, ou des mélanges de ceux-ci : 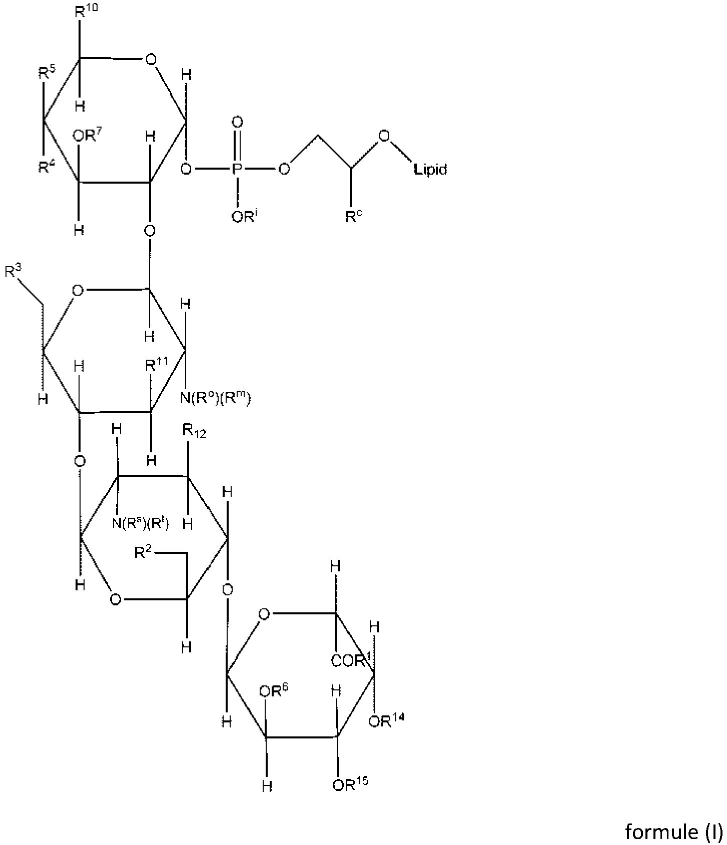 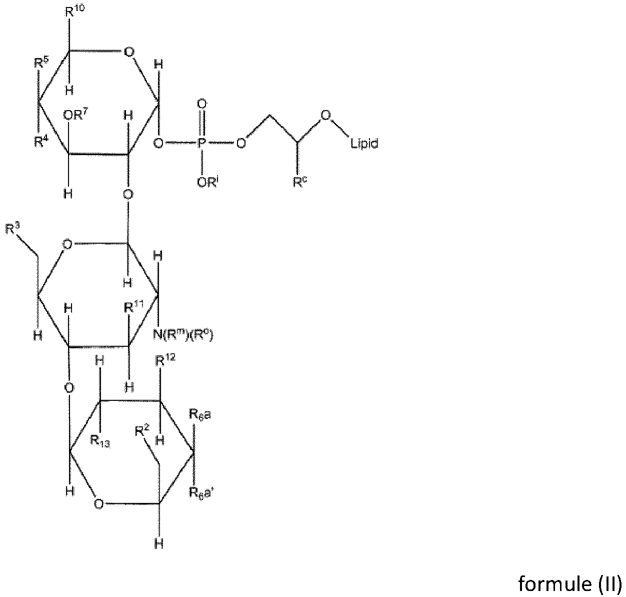 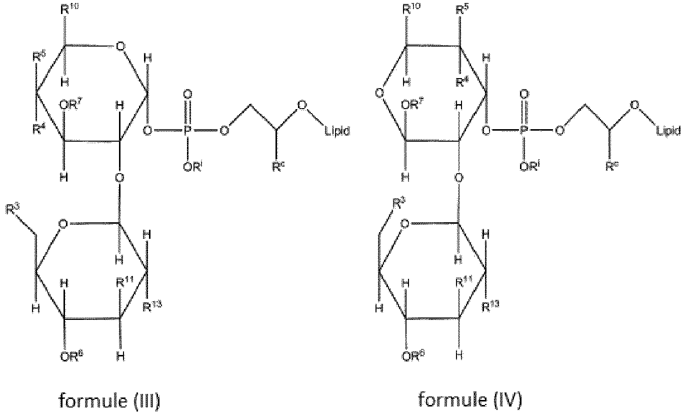 dans lequel
- chacun des R¹ est indépendamment sélectionné parmi -OH ou -N(R^{1a})(R^{1b}), dans lequel chacun de R^{1a} et R^{1b}, indépendamment l'un de l'autre et à chaque occurrence, est sélectionné parmi le groupe consistant en H, un groupe de protection amino, aryle, hétéroaryle, aliphatique et un groupe hétéroaliphatique,
- chacun de R², R⁴, R⁵, R¹¹ et R¹², indépendamment les uns des autres et à chaque occurrence, est sélectionné parmi le groupe consistant en H, -OR^{Z}, -N(R^{Z})₂, aryle, hétéroaryle, aliphatique et un groupe hétéroaliphatique, dans lequel R^{z} est indépendamment sélectionné parmi le groupe consistant en H, un groupe de protection hydroxyle, un groupe de protection amino, aryle, hétéroaryle, aliphatique, hétéroaliphatique et une fraction glucidique,
- chacun des R³ est indépendamment sélectionné parmi le groupe consistant en H, -OH, -NH₂, -SH, OR^{W}, - NH(R^{W}), -N(R^{W})₂, -SR^{W}, -O(C=O)R^{W}, -NH(C=O)R^{W}, -O(C=NH)R^{W}, -NH(C=NH)R^{W}, -S(C=NH))R^{W}, -NH(C=S)R^{W}, -S(C=O)R^{W}, -O(C=S)R^{W}, -S(=S)R^{W}, aryle, hétéroaryle, aliphatique, et un groupe hétéroaliphatique, dans lequel Rw est sélectionné parmi le groupe consistant en une fraction glucidique, aryle, hétéroaryle, aliphatique, et un groupe hétéroaliphatique,
- chacun de R⁶, R¹⁴ et R¹⁵, indépendamment les uns des autres et à chaque occurrence est sélectionné parmi le groupe consistant en H, un groupe de protection hydroxyle, aryle, hétéroaryle, aliphatique, un groupe hétéroaliphatique et une fraction glucidique,
- chacun des R⁷ est indépendamment sélectionné parmi le groupe consistant en H, C(=O)N(R^{z'})₂, -C(=O)OR^{z'}, un groupe de protection hydroxyle, aryle, hétéroaryle, aliphatique, un groupe hétéroaliphatique et une fraction glucidique, dans lequel R^{z'} est indépendamment sélectionné parmi le groupe consistant en H, un groupe de protection hydroxyle, un groupe de protection amino, aryle, hétéroaryle, aliphatique, et un groupe hétéroaliphatique,
- chacun des R¹⁰ est indépendamment sélectionné parmi le groupe consistant en -C(=O)N(R')(R^{p}), -C(=O)OR^{k}, et -CH₂OR^{k}, dans lequel R¹ et R^{p} sont sélectionnés indépendamment l'un de l'autre et à chaque occurrence parmi le groupe consistant en H, un groupe de protection amino, aryle, hétéroaryle, aliphatique, un groupe hétéroaliphatique et une fraction glucidique, dans lequel R^{k} est indépendamment sélectionné parmi le groupe consistant en H, un groupe de protection hydroxyle, aryle, hétéroaryle, aliphatique, un groupe hétéroaliphatique et une fraction glucidique,
- chacun des R¹³ est indépendamment sélectionné parmi le groupe consistant en -OH et -N(R^{o"})(R^{m"}), dans lequel R°^{"} et R^{m"} indépendamment l'un de l'autre et à chaque occurrence, sont sélectionnés parmi le groupe consistant en H, -C(=O)R^{h}, un groupe de protection amino, aryle, hétéroaryle, aliphatique et un groupe hétéroaliphatique, dans lequel R^{h} est sélectionné parmi le groupe consistant en une fraction glucidique, aryle, hétéroaryle, aliphatique et un groupe hétéroaliphatique,
- chacun de (R°) et (R^{m}), indépendamment l'un de l'autre et à chaque occurrence, est sélectionné parmi le groupe consistant en H, -C(=O)R^{W'}, un groupe de protection amino, aryle, hétéroaryle, aliphatique et un groupe hétéroaliphatique, dans lequel R^{w} est sélectionné parmi le groupe consistant en une fraction glucidique, aryle, hétéroaryle, aliphatique et un groupe hétéroaliphatique ;
- chacun de (R^{s}) et (R^{t}), indépendamment l'un de l'autre et à chaque occurrence, est sélectionné parmi le groupe consistant en H, -C(=O)R^{W}, un groupe de protection amino, aryle, hétéroaryle, aliphatique et un groupe hétéroaliphatique, dans lequel R^{w"} est sélectionné parmi le groupe consistant en une fraction glucidique, aryle, hétéroaryle, aliphatique et un groupe hétéroaliphatique ;
- chacun de R^{6a} et R^{6a'}, indépendamment l'un de l'autre et à chaque occurrence, est sélectionné parmi H ou -OH, de préférence R^{6a} est H et R^{6a'} est -OH ;
- chacun des R^{c} est indépendamment sélectionné parmi le groupe consistant en H, halogène, hétéroaryle, -OR^{q}, -N(R^{q})₂, -SR^{q}, NO₂, -NC, -CN, -N₃, -N(R^{q})=NR^{q}, -CHO,-C(=O)R^{q}, -C(=S)R^{q}, C(=NR^{q})R^{q}, - C(=O)OR^{q}, -C(=NR^{q})OR^{q}, -C(=NR^{q})N(R^{q})₂, -C(=O)N(R^{q})₂,-C(=S)OR^{q}, -C(=O)SR^{q}, -C(=S)SR^{q}, - P(=O)(OR^{q})₂, -S(=O)(OR^{q}), -S(=0)₂(OR^{q}), -P(=O)N(R^{q})₂,-P(=O)₂N(R^{q})₂, -C(=0)NR'S(=O)₂R^{q}, - S(=O)N(R^{q})₂ et -S(=O)₂N(R^{q})₂, dans lequel chacun des R^{q} est indépendamment sélectionné parmi le groupe consistant en H, aliphatique, hétéroaliphatique, aryle, hétéroaryle, et un groupe de protection hydroxyle,
- chacun des R¹ est indépendamment sélectionné parmi le groupe consistant en H, un groupe de protection hydroxyle, aliphatique, hétéroaliphatique, aryle, et hétéroaryle,
- chaque Lipide est indépendamment sélectionné parmi H ou une fraction aliphatique en C₁₋₃₀, dans lequel 0 à 10 motifs méthylène sont éventuellement remplacés par -O-, -NR^{x}-, -S-, -C(=O)-, -C(=NR^{x})-, -S(=O)-, - S(=O)₂-, -N=N-, -C=N-, -C(R^{y})=C(R^{y'})-, -N-O-, un arylène, ou une fraction hétéroarylène, dans lequel chacun des R^{x} est indépendamment sélectionné parmi le groupe consistant en H, aliphatique, hétéroaliphatique, aryle, hétéroaryle, ou un groupe de protection amino, et dans lequel chacun de R^{y} et R^{y'}, indépendamment l'un de l'autre et à chaque occurrence est sélectionné parmi le groupe consistant en H, aliphatique, hétéroaliphatique, aryle et un groupe hétéroaryle.

2. Composé pour utilisation selon la revendication 1, dans lequel ledit composé est au moins l'un des composés selon la formule générale (la), ou le sel pharmaceutiquement acceptable, le solvate pharmaceutiquement acceptable, ou un mélange de ceux-ci, 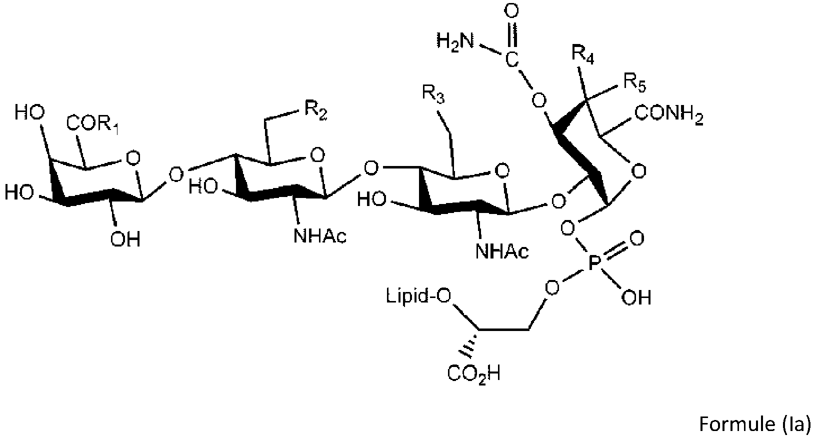 dans lequel
- chacun des R¹ est indépendamment sélectionné parmi OH, NH₂, ou NH(R^{1b}), dans lequel R^{1b} est un groupe cycloalkyle en C₁₋₁₀ ou cycloalcényle en C₁₋₁₀ qui est éventuellement substitué par un halogénure, hydroxyle ou alcoxy, plus préférentiellement R¹ est -NH(R^{1b}), dans lequel R^{1b} est de la formule suivante : 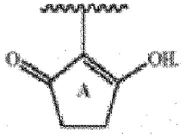
- chacun des R³ est indépendamment sélectionné parmi le groupe consistant en H, -OH et -OR^{W}, dans lequel R^{W} est D-glucose ;
- chacun de R² et R⁴, indépendamment l'un de l'autre et à chaque occurrence, est sélectionné parmi H ou -OH ;
- chacun des R⁵ est indépendamment sélectionné parmi le groupe consistant en H, -OH et -CH₃ ;
- le Lipide est choisi parmi la Formule L₁ ou la Formule L₂ suivantes :

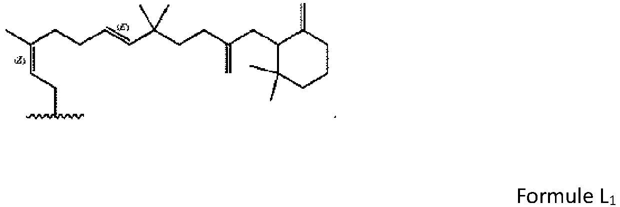

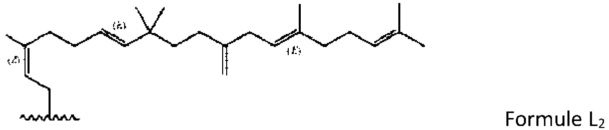

3. Composé pour utilisation selon la revendication 1 ou la revendication 2, dans lequel ledit composé est au moins l'un des composés selon la formule générale (Ib) ou le sel pharmaceutiquement acceptable, le solvate pharmaceutiquement acceptable, ou un mélange de ceux-ci,

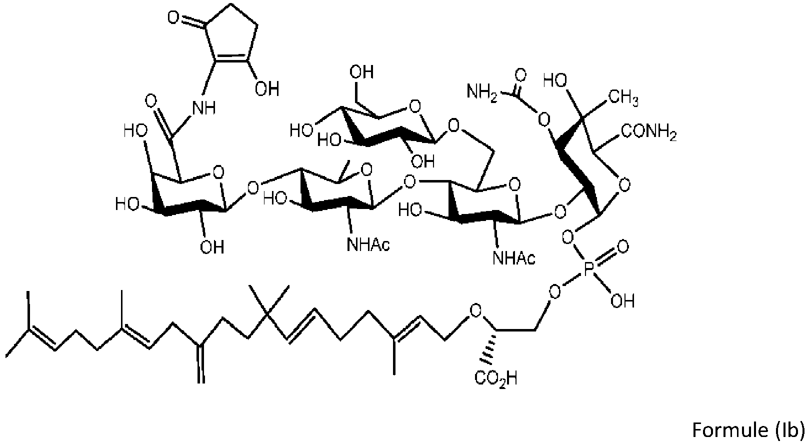

4. Composé pour utilisation selon la revendication 1, dans lequel ledit composé est au moins l'un des composés selon la formule (IIa) ou la formule (IIIa), ou le sel pharmaceutiquement acceptable, le solvate pharmaceutiquement acceptable, ou un mélange de ceux-ci : 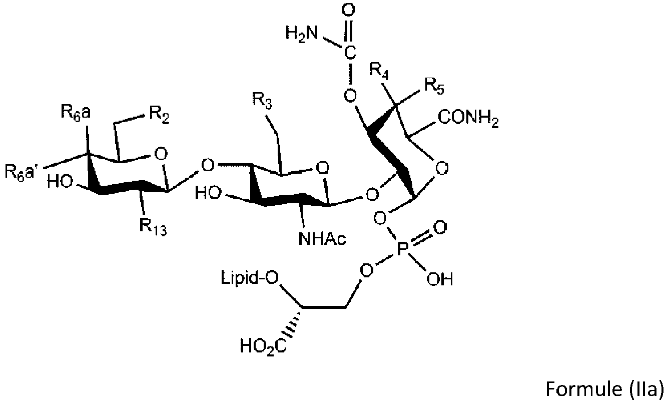 dans lequel
- R³est H ou -OH ;
- chacun de R² et R⁴, indépendamment l'un de l'autre et à chaque occurrence, est sélectionné parmi H ou -OH ;
- chacun de R^{6a} et R^{6a'}, indépendamment l'un de l'autre et à chaque occurrence, est sélectionné parmi H ou -OH, de préférence R^{6a} est H et R^{6a'} est -OH ;
- R⁵ est H, -OH ou CH₃ ;
- R¹³ est -OH ou -NHC(=O)CH₃
- le Lipide est choisi parmi la Formule L₃, la Formule L₄ ou la Formule L₅ suivantes : 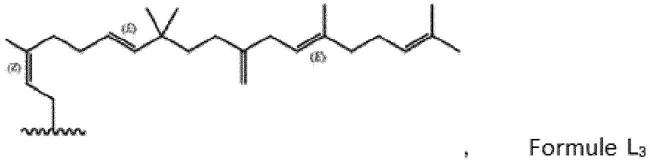 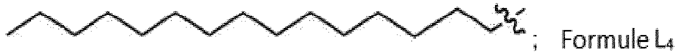 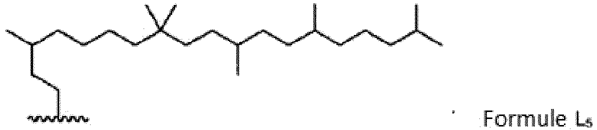
ou 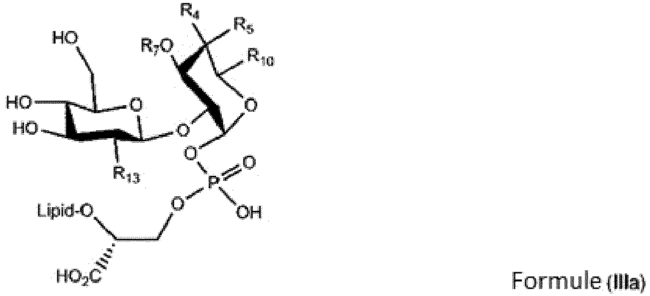 dans lequel
- chacun de R⁴ et R⁵ indépendamment l'un de l'autre et à chaque occurrence, est sélectionné parmi le groupe consistant en H, CH₃ et -OH ;
- R₇ est indépendamment sélectionné parmi H ou -C(=O)NH₂, de préférence, R₇ est C(=O)NH₂ ;
- R¹⁰ est indépendamment sélectionné parmi -C(=O)NH₂ ou -C(=O)OH, de préférence, R¹⁰ est C(=O)NH₂. ;
- R¹³ est indépendamment sélectionné parmi le groupe consistant en -OH ou - NHC(=O)CH₃, de préférence R¹³ est -NHC(=O)CH₃
- le Lipide est choisi parmi la Formule L₆, la Formule L₇ ou la Formule L₈ suivantes :

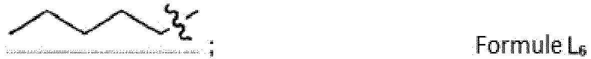

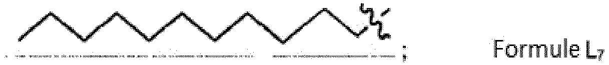

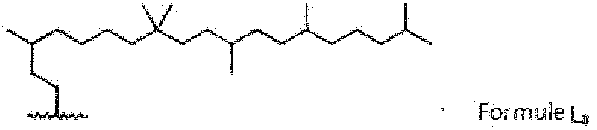

5. Composé pour utilisation selon l'une quelconque des revendications 1 à 4, dans lequel le traitement et/ou la prévention comprend l'administration d'une dose quotidienne de 0,5 à 100 mg du composé.

6. Composé pour utilisation selon l'une quelconque des revendications précédentes, dans lequel le traitement et/ou la prévention comprend l'administration d'une dose quotidienne de 0,5 à 3 mg/kg de poids corporel.

7. Composé pour utilisation selon l'une quelconque des revendications 1 à 6, dans lequel le composé est administré par voie orale.

8. Composé pour utilisation selon l'une quelconque des revendications précédentes, dans lequel le composé est administré au moins 10, de préférence au moins 14, jours de suite.

9. Composition pharmaceutique pour utilisation dans le traitement et/ou la prévention de l'EEL, la composition comprenant un composé selon l'une quelconque des revendications 1 à 4 et un ou plusieurs véhicules pharmaceutiquement acceptables.

10. Composition pharmaceutique pour utilisation selon la revendication 9, dans laquelle la composition comprend 0,05 à 100 mg du composé selon l'une quelconque des revendications 1 à 4.

11. Composition alimentaire pour lapins comprenant un composé selon l'une quelconque des revendications 1 à 4 pour utilisation dans le traitement et/ou la prévention de l'EEL.

12. Composition alimentaire pour lapins pour utilisation selon la revendication 11, dans laquelle la composition comprend d'environ 1 à environ 100 ppm du composé selon l'une quelconque des revendications 1 à 4.

13. Composition alimentaire pour lapins pour utilisation selon la revendication 11 ou la revendication 12, dans laquelle le traitement et/ou la prévention comprend l'administration par voie orale du composé selon l'une quelconque des revendications 1 à 4 dans la composition alimentaire pour lapins en dose quotidienne de 0,5 à 3 mg/kg de poids corporel pendant au moins 10, de préférence au moins 14, jours de suite.

14. Composition pharmaceutique ou alimentaire pour lapins pour utilisation selon l'une quelconque des revendications 9 à 13, dans laquelle la composition comprend de la bambermycine.
